(19) Europäisches Patentamt
European Patent Office
Office européen des brevets

(11) EP 1 874 304 B1

(12) **EUROPEAN PATENT SPECIFICATION**

(45) Date of publication and mention of the grant of the patent:
**15.07.2009 Bulletin 2009/29**

(21) Application number: **06754814.9**

(22) Date of filing: **24.04.2006**

(51) Int Cl.:
*A61K 31/4192* (2006.01) *A61P 3/04* (2006.01)
*C07D 403/06* (2006.01) *C07D 417/06* (2006.01)

(86) International application number:
**PCT/EP2006/061787**

(87) International publication number:
**WO 2006/117307 (09.11.2006 Gazette 2006/45)**

(54) **BENZOTRIAZOLE DERIVATIVES AS CANNABINOID RECEPTOR ANTAGONISTS**

BENZOTRIAZOLDERIVATE ALS CANNABINOIDREZEPTORANTAGONISTEN

DERIVES DE BENZOTRIAZOLE COMME ANTAGONISTES DU RECEPTEUR CANNABINOIDE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **29.04.2005 EP 05103597**

(43) Date of publication of application:
**09.01.2008 Bulletin 2008/02**

(73) Proprietor: **Janssen Pharmaceutica NV**
**2340 Beerse (BE)**

(72) Inventors:
- **BERWAER, Monique Jenny Marie**
**B-2340 Beerse (BE)**
- **LINDERS, Joannes Theodorus Maria**
**B-2340 Beerse (BE)**
- **KING, Peter John**
**B-2340 Beerse (BE)**
- **VAN HECKE, Geert Maria Robert**
**B-2340 Beerse (BE)**

(74) Representative: **Quaghebeur, Luc**
**Janssen Pharmaceutica N.V.,**
**Patent Department,**
**Turnhoutseweg 30**
**2340 Beerse (BE)**

(56) References cited:
**EP-A- 0 293 978** **US-A1- 2004 106 614**

- **LIDSTRÖM, P.; BONASERA, T.A., KIRILOVAS, D., LINDBLOM., B.; LU, L.; BERGSTRÖM, E.; BERGSTRÖM, M.; WESTLIN, J.; LANGSTRÖM, B.: "Synthesis, In Vivo Rhesus Monkey Biodistribution and In Vitro E valuation of a 11C-labelled Potent Aromatase Inhibitor: [N-methyl-11C]Vorozole" NUCLEAR MEDICINE & BIOLOGY, vol. 25, 1998, pages 497-501, XP002370103**
- **VENET, M.; WOUTERS, W., DE COSTER, R.; FREYNE, E.; SANZ, G.: "Vorozole, selective inhibitor of aromatase" ACTUALITES DE CHEMIE THERAPEUTIQUE, vol. 23, 1997, pages 239-246, XP008060736**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 874 304 B1

## Description

### Background of the invention

The present invention relates to a group ofbenzotriazole derivatives, to methods for the preparation of these compounds and to pharmaceutical compositions containing one or more of these compounds as active ingredient.

(1H-azol-1-ylmethyl)substituted benzotriazole derivatives have been described in EP 293 978; Venet M. et al., Actualités de chimie thérapeutique (1997) vol.23 p.239-246 and Lidström P. et al., Nuclear Medicine & Biology (1998) vol.25 p.497-501 as aromatase inhibitors useful for treating estrogen dependent disease. Recently fused tricyclic and tetracyclic pyrazole derivatives were described as potential $CB_1$ antagonists (Current Opinion in Drug Discovery & Development 2004 7(4):498-506).

It has now surprisingly been found that known and new benzotriazole derivatives of the formula (I) as well as the pharmaceutically acceptable addition salts and the stereoisomeric forms thereof, are potent cannabinoid $CB_1$ receptor modulators (known as antagonists or inverse agonists), and accordingly useful in the treatment obesity, psychiatric and neurological disorders, as well as other diseases involving cannabinoid-$CB_1$ neurotransmission (Current Opinion in Drug Discovery & Development 2004 7(4):498-506).

### Description of the invention

The present invention is concerned with benzotriazole derivatives of formula

(I)

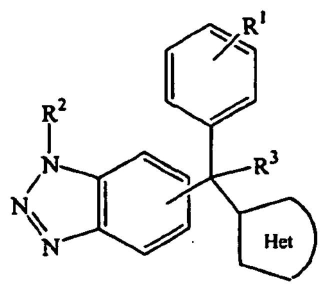

the pharmaceutically acceptable acid addition salts and stereoisomeric forms thereof, wherein

- $R^1$ is hydrogen, halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro;
- $R^2$ is hydrogen; $C_{1-10}$alkyl optionally substituted with $Ar^1$, $C_{3-7}$cycloalkyl, hydroxyl or $C_{1-6}$alkyloxy; $Ar^1$; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenoxy optionally substituted with $Ar^2$; $C_{2-6}$alkynyloxy; pyrimidinyloxy; di($Ar^2$)methoxy; (1-$C_{1-4}$alkyl-4-piperidinyl)oxy; or $R^2$ is $C_{1-10}$alkyloxy optionally substituted with halo; hydroxyl; $C_{1-6}$alkyloxy; amino; mono- and di($C_{1-6}$alkyl)amino; trifluoromethyl; carboxyl; $C_{1-6}$alkyloxycarbonyl; $Ar^1$; $Ar^2$-O-; $Ar^2$-S-; $C_{3-7}$cycloalkyl; 2,3-dihydro-1,4-benzodioxinyl; 1H-benzimidazolyl; $C_{1-4}$alkyl substituted 1H-benzimidazolyl; (1,1'-biphenyl)-4-yl or with 2,3-dihydro-2-oxo-1H-benzimidazolyl;
- $R^3$ is hydrogen, hydroxyl or $C_{1-6}$alkyl;
- $Ar^1$ is phenyl, naphthalenyl, pyridinyl, aminopyridinyl, imidazolyl, triazolyl, thienyl, halothienyl, furanyl, $C_{1-6}$alkylfuranyl, halofuranyl, thiazolyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, nitro or mono- and di- ($C_{1-6}$alkyl)amino;
- $Ar^2$ is phenyl, pyridinyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino) methyl, cyano, amino, mono- and di- ($C_{1-6}$alkyl)amino or nitro; and
- Het represents a monocyclic 5 or 6 membered partially saturated or aromatic heterocycle selected from furanyl, thienyl, pyrrolyl, oxazolyl, thiazolyl, imidazolyl, pyrazolyl, isoxazolyl, isothiazolyl, oxadiazolyl, triazolyl, thiadiazolyl, pyrimidinyl, pyridinyl, pyrazinyl, triazinyl, pyridazinyl, 2H-pyranyl or 4H-pyranyl wherein said heterocycle is optionally substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro; in a particular embodiment said Het is optionally substituted with

$C_{1-6}$alkyl.

In particular, to the use of the compounds of formula (I) as $CB_1$ receptor modulators, useful in the manufacture of a medicament for the treatment of obesity, psychiatric and neurological disorders, as well as other diseases involving cannabinoid-$CB_1$ neurotransmission. $CB_1$ receptor antagonists have potential in the treatment of a number of diseases such as neuroinflammatory disorders, cognitive and memory disorders, obesity, psychosis, gastrointestinal disorders and addiction (e.g. as an aid to smoking cessation). It has also been suggested that $CB_1$ receptor antagonists might be useful in the treatment of asthma following the discovery of pre-synaptic $CB_1$ receptor mediated effects on the inhibition of noradrenaline release in guinea pig lungs.

Another disease in which such compounds might have therapeutic potential is liver cirrhosis. This follows the observation of a reversal of low blood pressure in rats having $CCl_4$-induced liver cirrhosis, in conjunction with a lowering of mesenteric blood flow and portal vein pressure. It is accordingly an object of the present invention to provide the use of the compounds of formula (I) as $CB_1$ receptor modulators, useful in the manufacture of a medicament for the treatment of obesity, psychiatric and neurological disorders, as well as other diseases involving cannabinoid-$CB_1$ neurotransmission, in particular in the treatment of neuroinflammatory disorders such as for example Alzheimer's disease, Parkinson's disease, multiple sclerosis, HIV type-1 dementia, frontotemporal lobe dementia, and various prion diseases; cognitive and memory disorders such as for example dementia and schizophrenia; obesity; psychosis; addiction such as an aid to smoking cessation; and gastrointestinal disorders such as for example nausea and vomiting, gastric ulcers, irritable bowel syndrome, Chron's disease, secretory diarrhoea, paralytic ileus and gastroesophageal reflux.

As used in the foregoing definitions the term halo is generic to fluoro, chloro, bromo and iodo; the term "$C_{1-4}$alkyl" is meant to include straight and branch chained saturated hydrocarbon radicals having from 1 to 4 carbon atoms such as, for example, methyl, ethyl, 1-methylethyl, 1,1-dimethylethyl, propyl and the like; "$C_{1-6}$alkyl" is meant to include $C_{1-4}$alkyl radicals, as defined hereinabove, and the higher homologs thereof having from 5 to 6 carbon atoms such as, for example, 2-methylpropyl, butyl, pentyl, hexyl and the like; "$C_{1-10}$alkyl" is meant to include $C_{1-6}$alkyl radicals, as defined hereinabove, and the higher homologs thereof having from 7 to 10 carbon atoms; the term "$C_{3-7}$cycloalkyl" is generic to cyclopropyl, cyclobutyl, cyclopentyl, cyclohexyl and cycloheptyl; "$C_{2-6}$alkenyl" defines straight and branch chained hydrocarbon radicals containing one double bond and having from 2 to 6 carbon atoms such as, for example, ethenyl, 2-propenyl, 3-butenyl, 2-butenyl, 2-pentenyl, 3-pentenyl, 3-methyl-2-butenyl and the like; "$C_{2-6}$alkynyl" defines straight and branch chained hydrocarbon radicals containing one triple bond and having form 2 to 6 carbon atoms such as, for example, 2-propynyl, 2-butynyl, 3-butynyl, 2-pentynyl, 3-pentynyl, 4-pentynyl and the like; and when a $C_{2-6}$alkenyl or $C_{2-6}$alkynyl is substituted to a heteroatom, then the carbon atom of said $C_{2-6}$alkenyl or said $C_{2-6}$alkynyl connected to said heteroatom preferably is saturated.

The heterocycles as mentioned in the above definitions and hereinafter, are meant to include all possible isomeric forms thereof, for instance triazolyl also includes 1,2,4-triazolyl, and 1,3,4-triazolyl; oxadiazolyl includes 1,2,3-oxadiazolyl, 1,2,4-oxadiazolyl, 1,2,5-oxadiazolyl and 1,3,4-oxadiazolyl; thiadiazolyl includes 1,2,3-thiadiazolyl, 1,2,4-thiadiazolyl, 1,2,5-thiadiazolyl and 1,3,4-thiadiazolyl.

Further, the heterocycles as mentioned in the above definitions and hereinafter may be attached to the remainder of the molecule of formula (I) through any ring carbon or heteroatom as appropriate. Thus, for example, when the heterocycle is imidazolyl, it may be a 1-imidazolyl, 2-imidazolyl, 3-imidazolyl, 4-imidazolyl and 5-imidazolyl; when it is thiazolyl, it may be 2-thiazolyl, 4-thiazolyl and 5-thiazolyl.

Interesting compounds within the present invention are those compounds wherein the 1-Het-1-ylmethyl moiety is substituted on either the 5 or 6 position of the benzotriazole heterocyclic ring and wherein Het is a monocyclic 5 membered partially saturated or aromatic heterocycle selected from furanyl, thienyl, pyrrolyl, oxazolyl, thiazolyl, imidazolyl, pyrazolyl, isoxazolyl, isothiazolyl, oxadiazolyl, triazolyl or thiadiazolyl.

More interesting compounds within the invention are those interesting compounds of formula (I) wherein Het is imidazolyl or 1,2,4-triazolyl; $R^1$ is halo, $C_{1-4}$alkyl, $C_{1-4}$alkyloxy or trifluoromethyl; and $R^2$ is phenyl, $C_{3-7}$cycloalkyl or $C_{1-6}$alkyl optionally substituted with $Ar^1$.

Also of interest are those compounds wherein $R^2$ is hydrogen; $C_{1-6}$alkyl optionally substituted with phenyl, naphthalenyl, thienyl, furanyl, $C_{1-4}$alkylfuranyl, $C_{3-7}$cycloalkyl, hydroxyl or $C_{1-4}$alkyloxy; phenyl; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenyloxy optionally substituted with phenyl; $C_{2-6}$alkynyloxy; pyrimidinyloxy; di(phenyl)methoxy; (1-$C_{1-4}$alkyl-4-piperidinyl)oxy; or $C_{1-6}$alkyloxy optionally substituted with halo, hydroxyl, amino, mono- and di($C_{1-4}$alkyl)amino, trifluoromethyl, carboxyl, $C_{1-6}$alkyloxycarbonyl, phenyl, thienyl, furanyl, pyridinyl, phenoxy, phenylthio, $C_{3-7}$cycloalkyl, 2,3-dihydro-1,4-benzodioxinyl, 1H-benzimidazolyl, $C_{1-4}$alkyl substituted 1H-benzimidazolyl, (1,1-biphenyl)-4-yl, or with 2,3-dihydro-2-oxo-1H-benzimidazolyl.

A particular group of compounds are those compounds of formula (I) where one or more of the following restrictions apply;

- $R^1$ is hydrogen, halo, trifluoromethyl, $C_{1-4}$alkyl, $C_{1-4}$alkyloxy or $C_{1-4}$alkyloxycarbonyl-; in particular $R^1$ is halo, $C_{1-4}$alkyl, $C_{1-4}$alkyloxy or trifluoromethyl;
- $R^2$ is phenyl, $C_{3-7}$cycloalkyl or $C_{1-6}$alkyl optionally substituted with $Ar^1$; in particular $R^2$ is phenyl, cyclohexyl or $C_{1-6}$alkyl optionally substituted with $Ar^1$;
- $Ar^1$ is phenyl or phenyl substituted with up to 3 halo substituents; more in particular $Ar^1$ is phenyl or chloro-phenyl wherein the chloro substituent is at the para position vis-à-vis the attachment of the phenyl ring to the remainder of the molecule;
- Het represents a monocyclic 5 or 6 membered partially saturated or aromatic heterocycle selected from thiazolyl, imidazolyl, triazolyl, pyrimidinyl or pyridinyl wherein said heterocycle is optionally substituted with $C_{1-4}$alkyl; in a particular embodiment Het represents a monocyclic 5 membered partially saturated or aromatic heterocycle selected from thiazolyl, imidazolyl or triazolyl; in a more particular embodiment Het represents imidazolyl or 1,2,4-triazolyl.

A further group of compounds of formula (I), hereinafter referred to as the compounds of formula (Ia), are those that differ from the compounds disclosed in EP 293 978 and Lidström P. et al. *supra,* in that Het' does not represent imidazolyl; 1,2,4-triazolyl or 1,3,4-triazolyl. Venet M. et al. *supra,* discloses two compounds, i.e. 6-[(4-Chloro-phenyl)-pyridin-3-yl-methyl]-1-methyl-1H-benzotriazole and 6-[(4-Chloro-phenyl)-pyrimidin-5-yl-methyl]-1-methyl-1H-benzotriazole, wherein Het' represents pyridinyl and pyrimidinyl respectively, but said article is completely silent of the potential use of these compounds as CB-1 antagonists.

It is accordingly, an object of the present invention to provide the compounds of formula

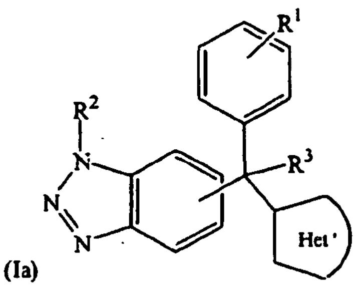

(Ia)

the pharmaceutically acceptable acid addition salts and stereoisomeric forms thereof, wherein

$R^1$ is hydrogen, halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro;

$R^2$ is hydrogen; $C_{1-10}$alkyl optionally substituted with $Ar^1$, $C_{3-7}$cycloalkyl, hydroxyl or $C_{1-6}$alkyloxy; $Ar^1$; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenoxy optionally substituted with $Ar^2$; $C_{2-6}$alkynyloxy; pyrimidinyloxy; di($Ar^2$)methoxy; (1-$C_{1-4}$alkyl-4-piperidinyl)oxy; or $R^2$ is $C_{1-10}$alkyloxy optionally substituted with halo; hydroxyl; $C_{1-6}$alkyloxy; amino; mono- and di($C_{1-6}$alkyl)amino; trifluoromethyl; carboxyl; $C_{1-6}$alkyloxycarbonyl; $Ar^1$; $Ar^2$-O-; $Ar^2$-S-; $C_{3-7}$cycloalkyl; 2,3-dihydro-1,4-benzodioxinyl; 1H-benzimidazolyl; $C_{1-4}$alkyl substituted 1H-benzimidazolyl; (1,1'-biphenyl)-4-yl or with 2,3-dihydro-2-oxo-1H-benzimidazolyl;

$R^3$ is hydrogen, hydroxyl or $C_{1-6}$alkyl;

$Ar^1$ is phenyl, naphthalenyl, pyridinyl, aminopyridinyl, imidazolyl, triazolyl, thienyl, halothienyl, furanyl, $C_{1-6}$alkylfuranyl, halofuranyl, thiazolyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, nitro or mono- and di- ($C_{1-6}$alkyl)amino;

$Ar^2$ is phenyl, pyridinyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino) methyl, cyano, amino, mono- and di- ($C_{1-6}$alkyl)amino or nitro; and

Het' represents a monocyclic 5 or 6 membered partially saturated or aromatic heterocycle selected from furanyl, thienyl, pyrrolyl, oxazolyl, thiazolyl, pyrazolyl, isoxazolyl, isothiazolyl, oxadiazolyl, thiadiazolyl, pyrimidinyl, pyridinyl, pyrazinyl, triazinyl, pyridazinyl, 2H-pyranyl or 4H-pyranyl wherein said heterocycle is optionally substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro; in a particular embodiment said Het ' is optionally substituted with $C_{1-6}$alkyl;

provided that said compound of formula (Ia) does not represent
6-[(4-Chloro-phenyl)-pyridin-3-yl-methyl]-1-methyl-1H-benzotriazole or
6-[(4-Chloro-phenyl)-pyrimidin-5-yl-methyl]-1-methyl-1H-benzotriazole.

A particular group of compounds are those compounds of formula (Ia) wherein one or more of the following restrictions apply;

- $R^1$ is hydrogen, halo, trifluoromethyl, $C_{1-4}$alkyl, $C_{1-4}$alkyloxy or $C_{1-4}$alkyloxycarbonyl-; in particular $R^1$ is halo, $C_{1-4}$alkyl, $C_{1-4}$alkyloxy or trifluoromethyl;
- $R^2$ is phenyl, $C_{3-7}$cycloalkyl or $C_{1-6}$alkyl optionally substituted with $Ar^1$; in particular $R^2$ is phenyl, cyclohexyl or $C_{1-6}$alkyl optionally substituted with $Ar^1$;
- $Ar^1$ is phenyl or phenyl substituted with up to 3 halo substituents; more in particular $Ar^1$ is phenyl or chloro-phenyl wherein the chloro substituent is at the para position vis-à-vis the attachment of the phenyl ring to the remainder of the molecule;
- Het ' represents a monocyclic 5 or 6 membered partially saturated or aromatic heterocycle selected from thiazolyl, pyrimidinyl or pyridinyl wherein said heterocycle is optionally substituted with $C_{1-4}$alkyl; in a particular embodiment Het ' represents thiazolyl;

Accordingly, the present invention relates to the compounds of formula (Ia) for use as a medicine, in particular to the use of the compounds of formula (Ia) in the manufacture of a medicament for the treatment of obesity, psychiatric and neurological disorders, as well as other diseases involving cannabinoid-$CB_1$ neurotransmission. In particular in the treatment of neuroinflammatory disorders such as for example Alzheimer's disease, Parkinson's disease, multiple sclerosis, HIV type-1 dementia, frontotemporal lobe dementia, and various prion diseases; cognitive and memory disorders such as for example dementia and schizophrenia; obesity; psychosis; addiction such as an aid to smoking cessation; and gastrointestinal disorders such as for example nausea and vomiting, gastric ulcers, irritable bowel syndrome, Chron's disease, secretory diarrhoea, paralytic ileus and gastroesophageal reflux.

The compounds of formula (I) wherein Het represents a 5 or 6 membered monocyclic partially saturated or aromatic *N*-comprising heterocycle, can generally be prepared as described in EP 293 978, i.e. by *N*-alkylating said heterocycle of formula (III) or an alkali metal salt thereof with a benzotriazole of formula (II).

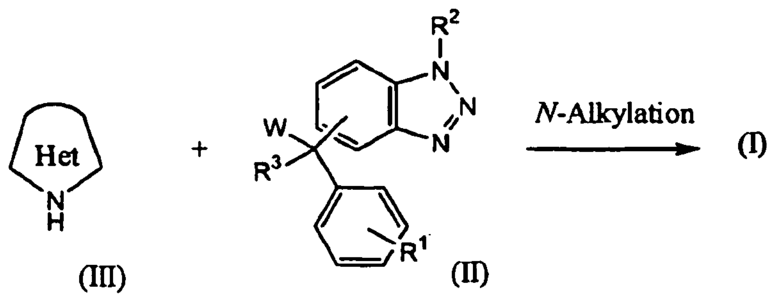


W as used in the reaction of (II) with (III) is an appropriate leaving group such as, for example, halo, e.g. chloro, a sulfonyloxy group, e.g. 4-methylbenzenesulfonyloxy.

The above described *N*-alkylation is conveniently carried out by stirring the reactants in the presence of a suitable organic solvent such as, for example, an aromatic hydrocarbon, e.g. methylbenzene; a ketone, e.g. 4-methyl-2-pentanone; an ether, e.g. tetrahydrofuran; a polar aprotic solvent, e.g., *N*,*N*-dimethylformamide; and mixtures of such solvents. Somewhat elevated temperatures may be appropriate to enhance the rate of the reaction.

The addition of an appropriate base such as, for example, an alkali or an earth alkaline metal carbonate, hydrogen carbonate, hydroxide, amide or hydride, e.g., sodium hydroxide or sodium hydride; or an organic base, such as, for example, pyridine or *N*,*N*-diethylethanamine may be employed.

Alternatively, the compounds of formula (I) wherein Het represents a 5 or 6 membered monocyclic partially saturated or aromatic *N*-comprising heterocycle, can be prepared by;

i) *N*-alkylating said heterocycle of formula (III) or an alkali metal salt thereof with an arylamine of formula (IV);

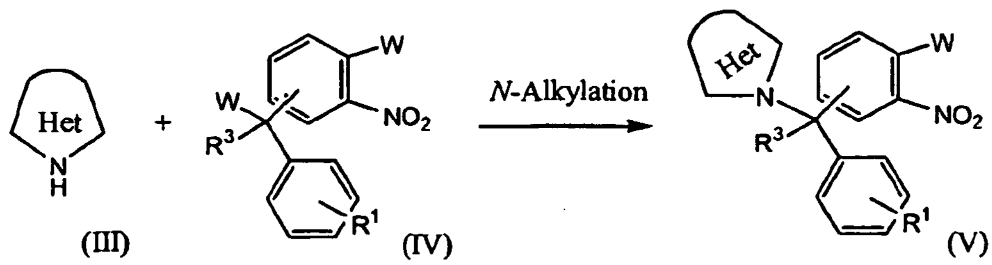

(III) (IV) (V)

ii) *N*-alkylating a secondary amine of formula (VI) with the compound of formula (V) to yield the nitroarylamine of formula (VII);

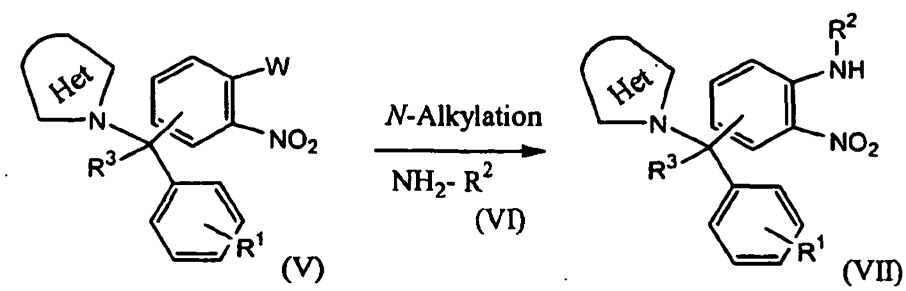

(V) (VII)

iii) followed by reduction and *N*-nitrosation to obtain the compounds of formula (I).

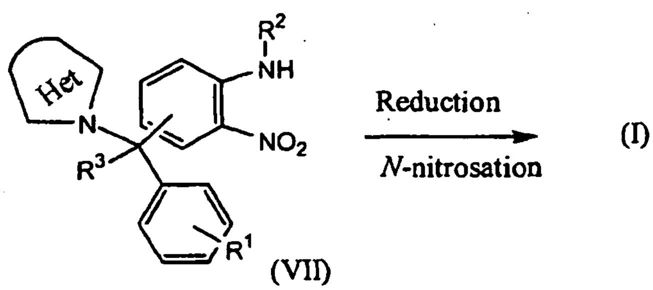

(VII)

The use of W in steps i) and ii) *supra* refers to an appropriate leaving group such as, for example, halo, e.g. chloro, a sulfonyloxy group, e.g. 4-methylbenzenesulfonyloxy. *N*-alkylation in steps i) and ii) *supra* is conveniently carried out as described hereinbefore.

The reduction of the aromatic nitro compound (VII) is conveniently carried out using art known reducing agents such as for example described in Advanced Organic Chemistry - Jerry March - third edition - section 9-48. Many reducing agents have been used to reduce aromatic nitro compounds, among them Zn, Sn or Fe and acid; catalytic hydrogenation such as Pt/C 5%; $AlH_3$-$AlCl_3$; hydrazine and a catalyst; dodecacarbonyltriiron[$Fe_3(CO)_{12}$]-methanol; $TiCl_3$; hot liquid paraffin; formic acid and Pd/C; and sulfides such as NaHS, $(NH_4)_2S$ or polysulfides. As described in the examples hereinafter, the preferred reduction reaction is a catalytic hydrogenation using Pt/C 5%.

The *N*-nitrosation reaction is done using art know procedures such as for example described in Advanced Organic Chemistry - Jerry March - third edition - section 2-50. The reaction is typically performed with nitrous acid generated *in situ* from sodium nitrite in an aqueous hydrochloric acid solution or in acetic acid.

The compounds of formula (I) wherein the 5 or 6 membered monocyclic partially saturated or aromatic *N*-comprising heterocycle (Het) is not attached via de *N*-atom to the remainder, hereinafter referred to as the compounds of formula (Ib), can generally be prepared as by alkylating the benzotriazole of formula (VIII) with said heterocycle of formula (III) as an organometallic reagent at low temperatures;

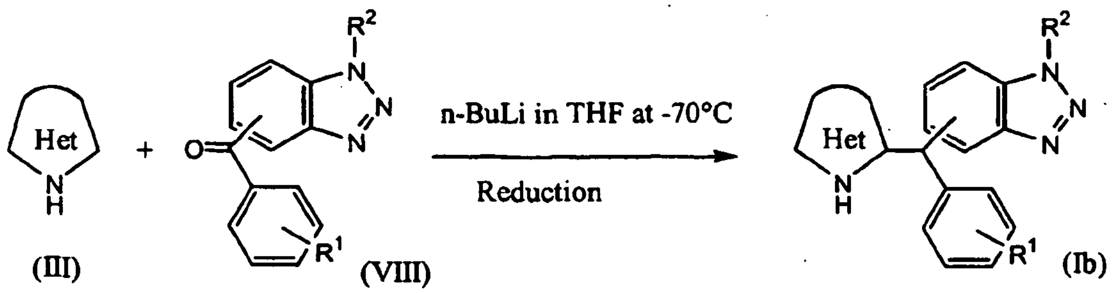


(III) (VIII) (Ib)

The above described alkylation is conveniently carried out by converting the heterocycle in a firs step into the aryllithium reagent by adding butyllithium to the heterocycle at a low temperature, typically-70°C, in the presence of a suitable organic solvent such as an ether, e.g. tetrahydrofuran. In the subsequent step the thus obtained organometallic reagent is stirred with the benzotriazole of formula (VIII) at a low temperature, typically -70°C in the same suitable organic solvent for 1 - 6 hours, typically 2 hours.

The thus obtained triarylcarbonol can easily be reduced with a hydrogenating agent such as $LiAlH_4$-$AlCl_3$; $NaBH_4$ in $F_3CCOOH$; diiodomethylsilane ($Me_2SiI_2$), $Fe(CO)_5$, $P_2I_4$; or tin and hydrochloric acid. As provided in the examples hereinafter, the triarylcarbonol is typically reduced using $SnCl_2$ and HCl (12N).

EP 293 978 further provides the preparation, formulation and pharmaceutical properties of aromatase inhibitors useful for treating estrogen dependent disease, of formula (Ic). The compounds of formula (Ic) are represented by

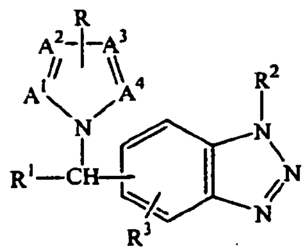

(Ic)

the pharmaceutically acceptable acid addition salts or a stereochemically isomeric form thereof, wherein $A^1=A^2-A^3=A^4$ is a bivalent radical having the formula

-CH=N-CH=CH- (a-1),

-CH=N-CH=N- (a2),

or

-CH=N-N=CH- (a3);

R is hydrogen or $C_{1-6}$alkyl;

$R^1$ is hydrogen, $C_{1-10}$alkyl, $C_{3-7}$cycloalkyl, $Ar^1$, $Ar^2$-$C_{1-6}$alkyl, $C_{2-6}$alkenyl or $C_{2-6}$alkynyl;

$R^2$ is hydrogen; $C_{1-10}$alkyl optionally substituted with $Ar^1$, $C_{3-7}$cycloalkyl, hydroxyl or $C_{1-6}$alkyloxy; $Ar^1$; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenoxy optionally substituted with $Ar^2$; $C_{2-6}$alkynyloxy; pyrimidinyloxy; di($Ar^2$)methoxy; (1-$C_{1-4}$alkyl-4-piperidinyl)oxy; or $R^2$ is $C_{1-10}$alkyloxy optionally substituted with halo; hydroxyl; $C_{1-6}$alkyloxy; amino; mono- and di($C_{1-6}$alkyl)amino; trifluoromethyl; carboxyl; $C_{1-6}$alkyloxycarbonyl; $Ar^1$; $Ar^2$-O-; $Ar^2$-S-; $C_{3-7}$cycloalkyl; 2,3-dihydro-1,4-benzodioxinyl; 1H-benzimidazolyl; $C_{1-4}$alkyl substituted 1H-benzimidazolyl; (1,1-biphenyl)-4-yl or with 2,3-dihydro-2-oxo-1H-benzimidazolyl;

$R^3$ is hydrogen, nitro, amino, mono- and di($C_{1-6}$alkyl)amino, halo, $C_{1-6}$alkyl, hydroxyl or $C_{1-6}$alkyloxy,

$Ar^1$ is phenyl, substituted phenyl, naphthalenyl, pyridinyl, aminopyridinyl, imidazolyl, triazolyl, thienyl, halothienyl, furanyl, $C_{1-6}$alkylfuranyl, halofuranyl or thiazolyl; and

$Ar^2$ is phenyl, pyridinyl or phenyl substituted with up to 3 substituents each independently selected from halo, hydroxyl,

hydroxymethyl, trifluoromethyl, $C_{1-6}$alkyl, $C_{1-6}$alkyloxy-, $C_{1-6}$alkyloxycarbonyl-, carboxyl, formyl, (hydroxyimino) methyl, cyano, amino, nitro or mono- and di- ($C_{1-6}$alkyl)amino;

Unexpectedly, we have now found that the aromatase inhibitors identified *supra*, which may hereinafter be referred to as compounds according to the present invention, are potent cannabinoid-$CB_1$ modulators (known as antagonists or inverse agonists), useful in the treatment obesity, psychiatric and neurological disorders, as well as other diseases involving cannabinoid-$CB_1$ neurotransmission. In particular in the treatment of neuroinflammatory disorders such as for example Alzheimer's disease, Parkinson's disease, multiple sclerosis, HIV type-1 dementia, frontotemporal lobe dementia, and various prion diseases; cognitive and memory disorders such as for example dementia and schizophrenia; obesity; psychosis; addiction such as an aid to smoking cessation; and gastrointestinal disorders such as for example nausea and vomiting, gastric ulcers, irritable bowel syndrome, Chron's disease, secretory diarrhoea, paralytic ileus and gastroesophageal reflux.

For one of the compounds disclosed in EP 293 978, i.e the racemate 1H-Benzotriazole, 6-[(4-chlorophenyl)-1H-1,2,4-triazol-1-ylmethyl]-1-cyclohexyl- it has now surprisingly been found that only one of the enantiomers, i.e. 1H-Benzotriazole, (-)-6-[(4-chlorophenyl)-1H-1,2,4-triazol-1-ylmethyl]-1-cyclohexyl- is active as a $CB_1$ modulator. It is accordingly an object of this invention to provide a compound according to formula (Ic) wherein said compound consists of the enantiomer *1H-Benzotriaiole, (-)-6-[(4-chlorophenyl)-1H-1,2,4-triazol-1-ylmethyl]-1-cyclohexyl-;* in particular said compound for use as a medicine, even more particular for use in the manufacture of a medicament for treating obesity, psychiatric and neurological disorders, as well as other diseases involving cannabinoid-$CB_1$ neurotransmission. Accordingly, the present invention relates to the use of compounds of formulae (I), (Ia), (Ib), (Ic) in the manufacture of a medicament for the treatment of obesity, psychiatric and neurological disorders, as well as other diseases involving cannabinoid-$CB_1$ neurotransmission as identified hereinbefore.

In particular, the present invention is concerned with the use of an aromatase inhibitor for the preparation of a pharmaceutical composition for treating inflammatory bowel disease, wherein said aromatase inhibitor is an (1H-azol-1-ylmethyl)substituted benzotriazole derivative of formula (I).

The compounds of formula (I) and some of the intermediates in this invention may have an asymmetric carbon atom in their structure. This chiral center may be present in a R- and a S-configuration.

Pure stereochemically isomeric forms of the compounds of this invention may be obtained by the application of art-known procedures. Diastereoisomers may be separated by physical separation methods such as selective crystallization and chromatographic techniques, e.g., counter current distribution, and enantiomers may be separated from each other by the selective crystallization of their diastereomeric salts with optically active acids. They may also be derived from the corresponding pure stereochemically isomeric forms of the appropriate starting materials, provided that the reaction occurs stereospecifically.

The compounds of formula (I), the pharmaceutically acceptable acid-addition salts and possible stereochemically isomeric forms thereof have very interesting pharmacological properties. They modulate the action of the cannabinoid-$CB_1$ receptor.

The cannabinoid receptors belong to the class G protein-coupled receptors and functional stimulation of $CB_1$ triggers, via activation of $G_{i/0}$ proteins, those intracellular signaling events that are normally coupled to these G protein-coupled receptors (GPCRs), that is:

(i) inhibition of stimulus-induced adenylate cyclase and subsequent impairment of cAMP/protein kinase A-mediated short- and long-term effects;
(ii) stimulation of mitogen-activated protein kinase signaling;
(iii) inhibition of voltage-gated, P, Q and *N*-type $Ca^{2+}$ channels and stimulation of inwardly rectifying G-protein-coupled $K^+$ channels and
(iv) stimulation of phosphatidylinositol 3-kinase and of intracellular $Ca^{2+}$ mobilization, seemingly through activation of PLC-$\gamma$, by the $\beta\gamma$ subunits of $G_{i/o}$ proteins.

It is based on these intracellular signaling effects that the modulation of the $CB_1$-receptor can be assessed in vitro, for example, by measuring the cAMP production in cells expressing the $CB_1$-receptor, such as for example, using a time-resolved fluorescence assay in which free cAMP contained in samples competes with an anti-cAMP Cryptate / cAMP-XL665 conjugate system. Alternatively, possible $CB_1$-receptor modulators can be identified using receptor binding assays either on membrane preparation or in situ on brain sections of lab animals, such as for example, provided in the examples hereinafter. The in vivo effect of the $CB_1$-receptor modulators, for example, be demonstrated by measuring the effect of the compounds in an acute dose-response on the food intake in male Sprague Dawley rats. The in vitro cAMP assay and $CB_1$ binding assays and the in vivo feeding experiment provided in the examples hereinafter, illustrate the capability of the compounds of formula (I) to modulate $CB_1$-receptor activity.

In view of their useful pharmacological properties, the subject compounds may be formulated into various

pharmaceutical forms for administration purposes.

To prepare the pharmaceutical compositions of this invention, an effective amount of the particular compound, in acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed.

The following examples are intended to illustrate and not to limit the scope of the invention. Unless otherwise stated all parts therein are by weight.

EXPERIMENTAL PART

Hereinafter, the term 'MP' means melting point, 'THF' means tetrahydrofuran, 'EtOAc' means ethyl acetate, 'DIPE' means diisopropyl ether, '$MgSO_4$' means magnesium sulphate, '$CH_2Cl_2$' means dichloromethane, 'DMA' means dimethylacetamide, 'DMSO' means dimethylsulfoxide, '$NaBH_4$' means sodium tetrahydroborate(-1).

A. Preparation of the intermediates

Example A1

a) Preparation of intermediate 1

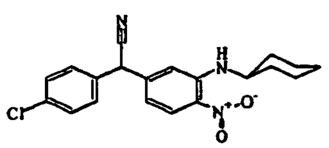

5-Chloro-*N*-cyclohexyl-2-nitrobenzenamine (0.244 mol) was stirred in THF (400 ml). Then, 4-chlorobenzeneacetonitrile (0.244 mol), a 10N sodium hydroxide solution (78.8 ml) and *N,N,N*-triethylbenzenememmaminium chloride (6.4 g) were added. The reaction mixture was stirred for 48 hours at 50°C. The reaction mixture was poured out into water, then it was extracted with $CH_2Cl_2$. The separated organic layer was dried ($MgSO_4$), filtered and the solvent evaporated. The residue was purified by column chromatography over silica gel (eluent: $CH_2Cl_2$). The desired fractions were collected and the solvent was evaporated, yielding 45 g (49.9%) of intermediate 1.

b) Preparation of intermediate 2

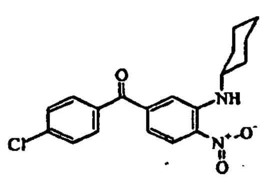

Intermediate 1 (0.07 mol) was stirred in DMA (250 ml). *N,N,N*-triethylbenzenemethanaminium chloride (1.3 g) was added, followed by potassium carbonate (13 g). Compressed air was allowed to bubble through during 48 hours

at room temperature. The reaction mixture was poured out into water. The resultant gum was filtered off, washed with water, then recrystallised from methanol. The precipitate was filtered off and dried, yielding 20.5 g (81.3%) of intermediate 2.

c) Preparation of intermediate 3

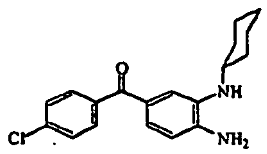

A mixture of intermediate 2 (0.07 mol) in methanol (250 ml) was hydrogenated at room temperature with Raney Nickel (25 g) as a catalyst. After uptake of $H_2$ (3 equiv, pressure: 3 bar), the catalyst was filtered off over Celite and the filtrate was evaporated, yielding 23 g of intermediate 3.

d) Preparation of intermediate 4

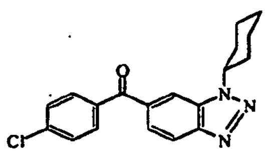

Intermediate 3 (0.07 mol) was stirred in a 6N HCl solution (250 ml) and cooled to 0˚C. A solution of sodium nitrite (0.104 mol) in water (20 ml) was added dropwise (at 0˚C) and the resultant reaction mixture was stirred for 2 hours at 0˚C. The mixture was alkalised with concentrated ammonia, then extracted with $CH_2Cl_2$. The separated organic layer was washed with water, dried ($MgSO_4$), filtered and the solvent evaporated, yielding 20 g (84%, oil) of intermediate 4.

e) Preparation of intermediate 5

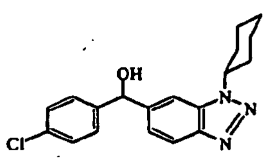

A solution of intermediate 4 (0.059 mol) in methanol (200 ml) was stirred at 0˚C. A solution of $NaBH_4$ (0.06 mol) in water (20 ml) was added and the resulting reaction mixture was stirred for one hour at 10˚C. The mixture was poured out into water and extracted with $CH_2Cl_2$. The separated organic layer was dried, filtered and the solvent evaporated. The residue (13 g of oil) was purified by column chromatography over silica gel (eluent: $CH_2Cl_2$/methanol 98/2). The product fractions were collected and the solvent was evaporated, yielding 8 g (40%) of intermediate 5.

f) Preparation of intermediate 6

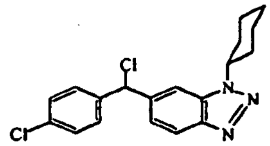

Thionyl chloride (8 ml) was added to intermediate 5 (0.023 mol) in $CH_2CH_2$ (100 ml), stirred at 0˚C. The reaction mixture was stirred for 12 hours at room temperature. The solvent was evaporated, yielding 8.5 g of intermediate 6.

g) Preparation of intermediate 7

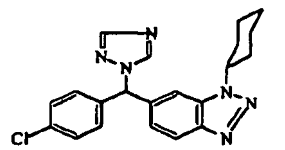

A mixture of intermediate 6 (0.023 mol), 1H-1,2,4-triazole (0.117 mol) and potassium carbonate (0.117 mol) in acetonitrile (200 ml) was stirred and refluxed for 12 hours. The solvent was evaporated until dry. The residue was taken up into water, then extracted with $CH_2Cl_2$. The separated organic layer was washed with water, dried ($MgSO_4$), filtered and the solvent evaporated. The residue (10 g, oil) was purified by high-performance liquid chromatography over silica gel (eluent: $CH_2Cl_2$/methanol 98/2). Several product fractions were collected and the solvent was evaporated. One fraction yielded 2.4 g and was recrystallised from diethyl ether, filtered off and dried, yielding 1.8 g (20%; MP: 154˚C) of intermediate 7.

<u>Example A2</u>

a) Preparation of intermediate 8

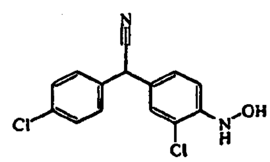

Potassium hydroxide (300 g) was stirred in methanol (1500 ml) (exothermic temperature rise to 70˚C). The mixture was allowed to cool to 55˚C. 4-Chlorobenzeneacetonitrile (1.38 mol) was added and the mixture was stirred for 15 min. A solution of 1-chloro-2-nitrobenzene (1.25 mol) in methanol (250 ml) was added (exothermic temperature rise to 50˚C). Water (2000 ml) was added and the mixture was stirred until it became an homogeneous suspension. The suspension was poured out into a mixture of ice (1250 g) and glacial acetic acid (545 ml). The mixture was stirred overnight. The resulting precipitate was filtered off and crystallised from 2-propanol. The precipitate was filtered off and then stirred in DIPE, filtered off and dried, yielding 80.5 g (22.0%, MP: 181.6˚C; (E+Z)) of intermediate 8.

b) Preparation of intermediate 9

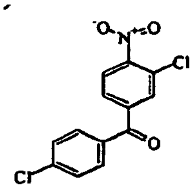

A mixture of intermediate 8 (0.172 mol), potassium hydroxide (120 g) and methanol (400 ml) in water (1200 ml) was stirred until an homogeneous red suspension was formed. A 30 % hydrogen peroxide (240 g) solution was added and the reaction mixture was stirred for 4 hours at 30-35°C. The yellow precipitate was filtered off, washed with water, then crystallised from ethanol. The precipitate was filtered off and dried, yielding 22 g (43.2%; MP: 120°C) of intermediate 9.

c) Preparation of intermediate 10

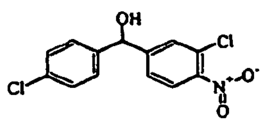

A mixture of intermediate 9 (0.1 mol) in methanol (250 ml) was stirred at 10°C. $NaBH_4$ (0.05 mol) was added portionwise. The mixture was stirred for 1 hour at 10°C, brought to room temperature and acetic acid (3 ml) in water (20 ml) was added dropwise. The mixture was evaporated, water (50 ml) was added and the product was extracted with DIPE. The organic layer was dried ($MgSO_4$) and evaporated, yielding : 27 g (90%) of intermediate 10.

d) Preparation of intermediate 11

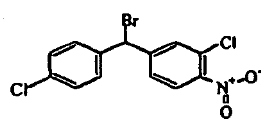

A 48% aqueous HBr (250 ml) solution was stirred and intermediate 10 (0.1 mol) was added portionwise. The mixture was refluxed for 2 hours and cooled. The product was extracted with toluene and washed with water. The organic layer was dried ($MgSO_4$) and evaporated, yielding 24 g (70%) of intermediate 11.

e) Preparation of intermediate 12

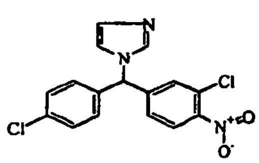

A mixture of intermediate 11 (0.0735 mol) and 1H-imidazole (0.397 mol) in acetonitrile (250 ml) was stirred and refluxed for 8 hours. The mixture was evaporated, water (200 ml) was added and extracted with $CH_2Cl_2$. The organic layer was extracted twice with HCl 1N. The aqueous layer was alkalised with ammonium hydroxide while cooling. The product was extracted with $CH_2Cl_2$. The organic layer was dried ($MgSO_4$) and evaporated. The free base residue was

converted into the ethanedioic acid salt (1:1) in 2-propanone. The precipitate was filtered off and dried, yielding 15 g (58%; .$C_2H_2O_4$ (1:1)) of intermediate 12.

f) Preparation of intermediate 13

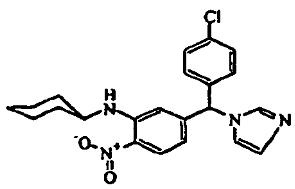

A mixture of the free base (0.047 mol) of intermediate 12 and cyclohexanamine (0.25 mol) in DMSO (62.5 ml) was stirred at 80˚C for 24 hours. Water was added and extracted with $CH_2Cl_2$. The organic layer was dried ($MgSO_4$) and evaporated. The residue (28.8 g) was purified on a glass filter over silica gel and by column chromatography over silica gel (eluent: methanol/trichloromethane 2/98). The pure fractions were collected and evaporated. The residue was evaporated again with toluene. The obtained residue (2.0 g) was converted into the ethanedioic acid salt (1:1) in 2-propanol. The product was precipitated with DIPE and decanted off. The residue was crystallised from 2-propanone. The precipitate was filtered off, washed with 2-propanone and dried in vacuo at 50˚C, yielding 0.2 g (MP: 114.4˚C; $C_2H_2O_4$ (1:1)) of intermediate 13.

g) Preparation of intermediate 14

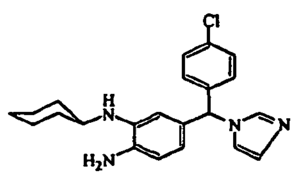

A mixture of the free base (0.026 mol) of intermediate 13 in a 4% thiophene solution (2 ml) and methanol (250 ml) was hydrogenated at room temperature with Pt/C (5%) (2 g) as a catalyst. After uptake of $H_2$ (3 equivalents) the catalyst was filtered off and the filtrate was evaporated. The residue was evaporated again with toluene, yielding 7.9 g (77%) of intermediate 14.

Example A3

a) Preparation of intermediate 15

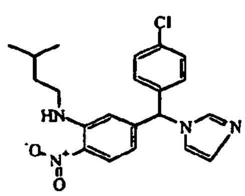

A mixture of the free base (0.047 mol) of intermediate 12 and 3-methyl-1-butanamine (0.24 mol) in DMSO (62.5 ml) was stirred at 80˚C for 24 hours. Water was added and extracted with $CH_2Cl_2$. The organic layer was dried ($MgSO_4$) and evaporated. The residue (25.5 g) was purified on a glass filter over silica gel (eluent: trichloromethane). The pure fractions were collected and evaporated. The residue (15.9 g) was purified by column chromatography over

silica gel (eluent : methanol/trichloromethane 2/98) The pure fractions were collected and evaporated. The obtained residue (2.8 g) was converted into the nitric acid salt (1:1) in EtOAc (10 ml). The product was precipitated with DIPE and decanted off. The residue was crystallised from EtOAc (15 ml). The precipitate was filtered off, washed with EtOAc, DIPE and dried in vacuo at 60˚C, yielding 2.1 g (69%; MP: 150.5˚C; .$HNO_3$ (1:1)) of intermediate 15.

b) Preparation of intermediate 16

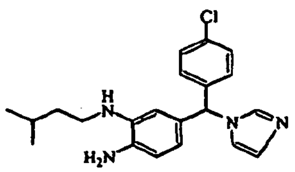

A mixture of the free base (0.015 mol) of intermediate 15 in a 4% thiophene solution (1 ml), methanol (200 ml) and $NH_3$/methanol (50 ml) was hydrogenated at room temperature with Pt/C (5%) (1 g) as a catalyst. After uptake of $H_2$ (3 equivalents) the catalyst was filtered off and the filtrate was evaporated. The residue was evaporated again with toluene, yielding 9.6 g (100%) of intermediate 16.

<u>Example A4</u>

a) Preparation of intermediate 17

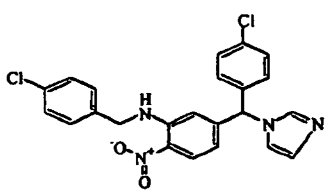

A mixture of the free base (0.047 mol) of intermediate 12 and 4-chlorobenzenemethanamine (0.21 mol) in DMSO (62.5 ml) was stirred at 80˚C for 24 hours. Water was added and extracted with $CH_2Cl_2$. The organic layer was dried ($MgSO_4$) and evaporated. The residue was purified on a glass filter over silica gel (eluent 1: trichloromethane) (eluent 2: (methanol/$NH_3$)/trichloromethane 2.5/97.5). The pure fractions were collected and evaporated. The residue was purified by column chromatography over silica gel (eluent: (methanol/$NH_3$)/trichloromethane 1/99). The pure fractions were collected and evaporated, yielding 16.5 g (78%) of intermediate 17.

b) Preparation of intermediate 18

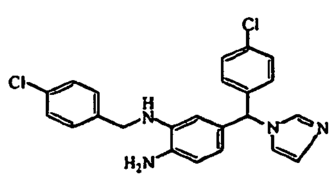

Water (250 ml) in sodium hydrosulfite (0.18 mol) was added to a stirring mixture of intermediate 17 (0.036 mol) in ethanol (600 ml) at room temperature. The mixture was stirred for 2 hours and evaporated. The residue was stirred in water and alkalised with sodium carbonate. The product was extracted with $CH_2Cl_2$. The organic layer was dried ($MgSO_4$) and evaporated, yielding 7.8 g (50%) of intermediate 18.

Example A5

a) Preparation of intermediate 19

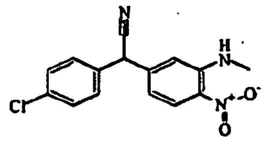

4-Chlorobenzeneacetonitrile (0.562 mol) was added to 5-chloro-*N*-methyl-2-nitrobenzenamine (0.536 mol) in THF (620 ml). *N,N,N*-triethylbenzenemethanaminium chloride (14 g) was added. 10N Sodium hydroxide (173.2 ml) was added and the resulting reaction mixture was stirred for 48 hours at 50 ˚C. The mixture was poured out into water, acidified with concentrated hydrochloric acid, then extracted with EtOAc. The separated organic layer was washed with water, dried ($MgSO_4$), filtered and the solvent evaporated. The residue was stirred in DIPE, filtered off, washed with DIPE, then dried, yielding 108 g (66.8%) of intermediate 19.

b) Preparation of intermediate 20

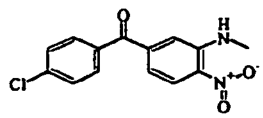

Intermediate 19 (0.358 mol) was stirred in DMA (800 ml). Potassium carbonate (54 g) and *N,N,N*-triethylberoenemethanaminium chloride (5.4 g) were added. Compressed air ($O_2$) was allowed to bubble through the mixture for 48 hours at room temperature. The mixture was poured out into water. The whole was filtered and the filter residue was washed with water, then taken up into DIPE (3 x), filtered off and dried, yielding 100 g (97%) of intermediate 20.

c) Preparation of intermediate 21

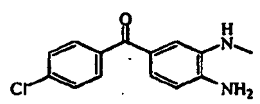

A mixture of intermediate 20 (0.206 mol) in methanol (500 ml) was hydrogenated for 2 hours with Raney Nickel (50 g) as a catalyst. After uptake of $H_2$ (3 equivalents), the catalyst was filtered off and the filtrate was evaporated, yielding 38 g (70.6%) of intermediate 21.

d) Preparation of intermediate 22

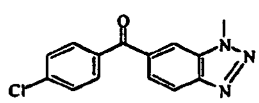

Intermediate 21 (0.146 mol) was stirred in 6N HCl (400 ml) and cooled to 5˚C. A solution of sodium nitrite (0.218 mol) in water (q.s.) was added, while the temperature was kept at 5˚C. Then, the reaction mixture was stirred for 6 hours

at room temperature. The precipitate was filtered off and dried, yielding 32 g (80.8%; MP: 169.9°C) of intermediate 22.

e) Preparation of intermediate 23

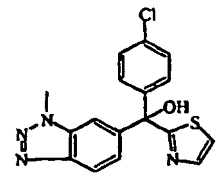

n-Butyllithium (29.3 ml) was added dropwise to a solution of thiazole (0.0375 mol) in THF at -70°C under $N_2$ and the mixture was stirred at -70°C for 1 hour. A solution of intermediate 22 (0.0312 mol) in THF was added dropwise and the mixture was stirred at -70°C for 2 hours. The mixture was quenched with water and extracted with diethyl ether. The organic layer was dried ($MgSO_4$), filtered off and evaporated. The residue (11.3 g) was crystallised from $CH_2Cl_2$, yielding 10.88 g (98%; MP: 162.1°C) of intermediate 23.

B. Preparation of the compounds

Example B1

Preparation of compounds 1 and 2

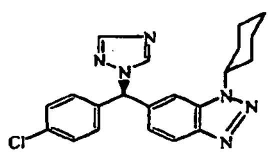

compound 1

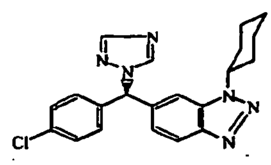

compound 2

Intermediate 7 (0.350 g, 0.00089 mol) was separated and purified by chiral column chromatography over ChiralPak AD (500 g, eluent: 100 % ethanol; flow: 110 ml/min). Two product fraction groups were collected and their solvent was evaporated, yielding 0.120 g ( $[\alpha]_{365}^{20^\circ C} = +15$ **(c: 1 mg/ml, methanol)**) of compound 1 and 0.120 g ( $[\alpha]_{365}^{20^\circ C} = -17$ **(c: 1 mg/ml, methanol)**) of compound 2.

Following compounds were made accordingly,

| | |
|---|---|
| 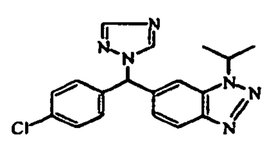 | 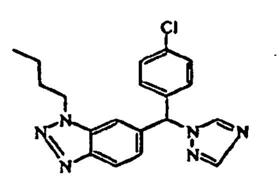 |
| Co. No. 7; $.C_2H_2O_4$ (1:1) | Co. No. 8; $.C_2H_2O_4$ (1:1); MP: 122.6°C |

Example B2

Preparation of compound 3

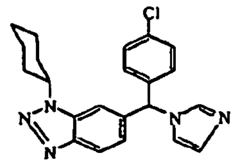

Intermediate 14 (0.021 mol) in 5N HCl (100 ml) was stirred at 0-5˚C. Sodium nitrite (0.105 mol) in water (20 ml) was added dropwise and the mixture was stirred at room temperature for 3 hours. The mixture was poured on ice and alkalised with sodium carbonate. The product was extracted with $CH_2Cl_2$. The organic layer was dried ($MgSO_4$) and evaporated. The residue (8.9 g) was purified twice on a glass filter over silica gel (eluent 1: methanol/trichloromethane 2/98) (eluent 2: (methanol-$NH_3$)/trichloromethane 1/99). The pure fractions were collected and evaporated. The residue (7.2 g) was crystallised from EtOAc (40 ml). The precipitate was filtered off, washed with EtOAc, DIPE and dried in vacuo at 50˚C, yielding 4.8 g (59 %; MP: 162.0˚C) of compound 3.

Following compounds were made accordingly,

| | |
|---|---|
| 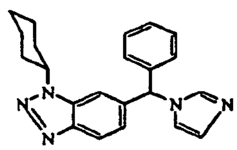 | 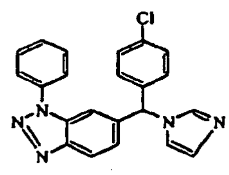 |
| Co. No. 9 | Co. No. 10; .$HNO_3$ (1:1); MP: 166.7˚C |
| 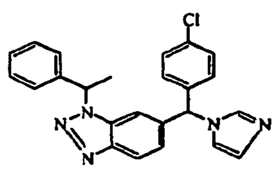 | 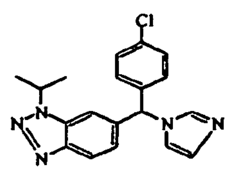 |
| Co. No. 11; .$HNO_3$ (1:1); MP: 160.7˚C | Co. No. 12; MP: 130.9 ˚C |
| 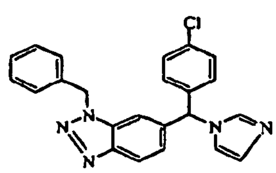 | |
| Co. No. 13; .HCl (1:1); MP: 188.6 ˚C | |

Example B3

Preparation of compound 4

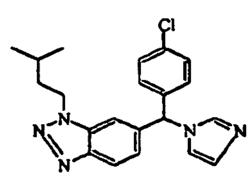

Intermediate 16 (0.026 mol) in 5N HCl (100 ml) was stirred at 0-5°C. Sodium nitrite (0.13 mol) in water (20 ml) was added dropwise and the mixture was stirred at room temperature overnight. The mixture was poured on ice and alkalised with sodium carbonate. The product was extracted with $CH_2Cl_2$. The organic layer was dried ($MgSO_4$) and evaporated. The residue (8.4 g) was purified on a glass filter over silica gel (eluent: methanol/trichloromethane 2/98). The pure fractions were collected and evaporated. The residue (7.4 g) was converted into the hydrochloric acid salt (1: 1) in EtOAc (20 ml). The product was precipitated with DIPE and decanted. The residue was crystallised from 2-propanone (15 ml). The precipitate was filtered off, washed with 2-propanone, DIPE and dried in vacuo at 50°C, to give 4.5 g (41.5%; MP: 163.8°C; .HCl (1:1)) of compound 4.

Example B4

Preparation of compound 5

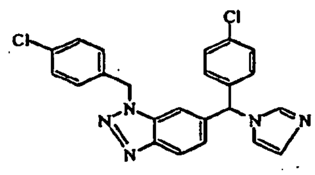

Intermediate 18 (0.0185 mol) in 5N HCl (100 ml) and acetic acid (35 ml) was stirred at 0-5°C. Sodium nitrite (0.0204 mol) in water (20 ml) was added dropwise and the mixture was stirred at room temperature for 2 hours. The mixture was poured on ice and alkalised with sodium carbonate. The product was extracted with $CH_2Cl_2$. The organic layer was dried ($MgSO_4$) and evaporated. The residue (7.2 g) was purified twice by column chromatography over silica gel (eluent 1: methanol/$CH_2Cl_2$ 5/95) (eluent 2: (methanol/$NH_3$)/EtOAc 2.5/97.5). The pure fractions were collected and evaporated. The residue (4.5 g) was purified on a glass filter over silica gel (eluent: (methanol/$NH_3$)/EtOAc 2.5/97.5). The pure fractions were collected and evaporated. The residue (4 g) was converted into the nitric acid salt (1:1) in EtOAc (20 ml). The product was precipitated with DIPE and decanted off. The residue was crystallised from 2-propanone (25 ml). The precipitate was filtered off, washed with DIPE and dried in vacuo at 60°C, yielding 3.5 g (38%; MP: 185.9°C; .$HNO_3$ (1:1)) of compound 5.

Example B5

Preparation of compound 6

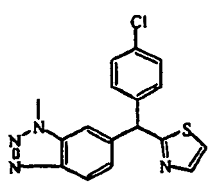

A mixture of intermediate 23(0.0248 mol) and tin chloride (28.25 g) in acetic acid (40 ml) and 12N HCl (40 ml) was stirred and refluxed overnight. The mixture was cooled, poured into ice water and basified with ammonium hydroxide. The mixture was filtered through celite and extracted with $CH_2Cl_2$. The organic layer was washed with a 10% potassium carbonate solution and water, dried ($MgSO_4$), filtered off and evaporated. The residue (6.45 g) was purified by column chromatography over silica gel (eluent : $CH_2Cl_2$/methanol/ammonium hydroxide 99.25/0.75/0.1) (15-40μm). The pure fractions were collected and evaporated. The residue (4.21 g) was converted into the nitric acid salt (1: 1) and recrystallised from methanol/diethyl ether, yielding : 2.54 g (50%; MP: 113.3°C; .$HNO_3$ (1:1)) of compound 6.

C. Pharmacological Examples

The capability of the compounds of the present invention to modulate $CB_1$-receptor activity, can be demonstrated

in the following test procedures.

*Reagents*

CP-55,940 [Side chain 2,3,4(N)-$^3$H]- (168 Ci/mmol) and [$^3$H]-microscales were purchased from PerkinElmer Life Sciences, Inc. (Boston, MA, USA) and Amersham Biosciences Europe GmbH (Benelux, Roosendaal, Nederland) respectively.
CP55,940, JWH133, Anandamide were purchased from Tocris Cookson (Bristol, UK). Sanofi's Rimonabant and JNJ compounds were obtained 'in-house' from central pharmacy. All other reagents were of high purity and obtained from the either Merck (Darmstadt, Germany) or Sigma-Aldrich NV/SA (Bomem, Belgium).

*Cells*

Human Cb-1 transfected CHO-K1 cells (Euroscreen; Cat # ES-110-C; hCB$_1$-D1; accession n° Swissprot X54937) were maintained as described previously (Felder et al., 1995 & 1998). For membrane preparation, cells were cultured in DMEM/Ham's F12 medium containing 10 % (v/v) heat inactivated FCS, 100 U/ml penicillin and 100 μg/ml streptomycin, 100 μg/ml pyruvate and 292 μg/ml L-glutamine under 5 % $CO_2$ at 37°C in roller bottles, with medium changed 3 times a week. When 90 % confluent, cells collected by trypsinisation and re-seeded at 1:10 or 1:20 dilutions to fresh roller bottles. For measurement of cAMP formation, cells were cultured to 90 % confluency in Falcon T175 flasks.

*Membrane preparation*

Prior to membrane preparation, confluent cells were treated with 5 mM butyrate for 24 hours. Medium was aspirated and cells were scraped from bottles in 50 ml homogenization buffer consisting of 15 mM Tris-HCl, pH 7.4 containing 2 mM $MgCl_2$, 0.3 mM EDTA and 1 mM EGTA. The cells were then centrifuged for 10 minutes at 1700 × g at 4 °C and homogenized in 20 ml same buffer with an Ultra Turrax. The crude membrane pellet was collected by two consecutive centrifugation steps at 20,000 × g for 10 min and for 20 min at 25,000 × g respectively, separated by a washing/homogenization step in 10 ml homogenization buffer. Final cell pellets were resuspended in a volume of 6 ml/ roller bottle in stock buffer consisting of 7.5 mM Tris-HCl, pH 7.4, containing 12.5 mM $MgCl_2$, 0.3 mM EDTA, I mM EGTA and 250 mM sucrose. Protein content was assayed by the BioRad method (Bradford, 1976).

*Animals*

Male Sprague Dawley rats (weighing 250-300 g at the time of experiment) were obtained from Charles River (Sulzfeld, Kisslegg, Germany). Male C57B1/6J Rj mice (weighing 25-30 g at the time of experiments) were obtained from Janvier (Le Genest-St-Isle, France). All animals had free access to water and were individually housed under 12-h light:dark cycle (lights on at 22:00 h) and at a temperature of 19-22 °C and 35-40 % humidity in Techniplast IVC cages adapted to fit external food hoppers. Rats and mice were fed a standard purified diet containing 10 % kcal fat (Dyets, Inc. Bethlehem, USA; or Research diets, New Brunswick, NJ, USA). All experiments were carried out in accordance with the European Communities Council Directives (86/609/EEC) and were approved by the local ethical committee.

<u>Example C1</u>: <u>Cb-1 competition receptor binding in cell membranes</u>

Competition binding assays were performed in triplicate in a final volume of 0.5 ml containing incubation buffer (50 mM Tris-HCl, pH 7.4 containing 2.5 mM EDTA and 0.5 % (w/v) BSA), 50 μl $^3$H-CP55,940 (0.5 nM final), 0.4 ml transfected CHO-K1 membrane proteins (60 μg/ml) in the presence or absence of test compound. Non-specific binding determined using 1 μM CP55,940. The samples were incubated for 1 hour at 25 °C, being terminated by rapid filtration on GF/C filters presoaked in 0.1 % polyethylenimine, using a Unifilter-96 Harvester (PerkinElmer N.V./S.A. Belgium). Filters were washed 6 times with cold washing buffer (50 mM Tris-HCl, pH 7.4 containing 0.1 % BSA), plates air-dried overnight before bound [$^3$H]CP55,940 was measured by liquid scintillation counting using a TopCount NXT microplate scintillation counter (Packard BioScience/ PerkinElmer N.V./S.A. Belgium). $IC_{50}$ values were determined by a single-site binding equation (GraphPad Prism, San Diego, CA, U.S.A.).

<u>Example C2</u>: <u>Cyclic AMP accumulation</u>

Using the commercially available dynamic homogenous time-resolved fluorescence cAMP assay, the cAMP measurements on CHO-K1 cells that stably express the Cb-1 receptor were performed. Cells were detached from flasks using 3 ml EDTA (0.04 % (w/v) in PBS) and resuspended in PBS (without $Ca^{2+}$ and $Mg^{2+}$) and centrifuged at 500 × g

for 5 min. Cell pellet was resuspended in stimulation buffer (HBSS medium containing 1 mM IBMX, 5 mM Hepes, 10 mM $MgCl_2$ and 0.1 % (w/v) BSA) and were dispensed into a black 96 well plate at a cell density of 20000 cells per well. After 30 min at 25 °C, an equal volume of stimulation buffer containing forskolin and CP55940 (agonist) and/or a Cb-1 antagonist was added to the cells. After 30 min incubation at 25 °C, cAMP detection was performed by addition of an equal volume of cAMP-XL665 and anti-cAMP-cryptate conjugates. The plate was incubated for a further 60 min at 25 °C and then measured with Discovery (PerkinElmer N.V./S.A. Belgium).

Example C3: Feeding experiments

An acute dose-response analysis for each compound was performed. All rats and mice were randomly assigned and orally dosed with either vehicle (10 % cyclodextran containing 0.9 % (w/v) saline) or vehicle containing a single concentration of compound (0.16, 0.63, 2.50, 10.0, 40.0 mg/kg at 10 ml/kg) so that the average latency from time of drug administration and lights out was 45 minutes, with 6 animals per group. Food intake was measured 1, 2, 4, 6 and 24 hours after lights out.

Example C4: Receptor occupancy experiments

In complementary experiments, for determining the occupancy of Cb-1 receptors by various compounds in different brain regions, rats and mice were treated with five doses ranging from 0.6 to 40 mg/kg under the same conditions as previously described. The animals were killed by decapitation 1 or 2 hr after lights out. Brains were quickly removed and frozen in 2-methylbutane cooled on dry ice (between -30 and -40 °C). Coronal sections (10 μm thick) were cut using a Reichert Jung 2800R cryostat-microtome (Cambridge Instruments, Cambridge, UK) were cut at the level of the striatum/ nucleus accumbens, anterior hypothalamus and mid-hypothalamus (0.70, -1.80, 3.30 mm rostral to Bregma, respectively) using the stereotaxic atlas (Paxinos & Watson, 1998) for anatomical reference, and thaw-mounted on polylysine-coated microscope slides (StarFrost, Knittel Gläser, Germany). The sections were stored at -80 °C until use.

*In vitro receptor binding*

This method has been previously described (Glass et al, 1997; Adams et al., 1998; Harrold et al., 2002). Saturation and association experiments were first performed to determine the $K_d$ of [$^3$H]CP55,940. Coronal sections containing the nucleus accumbens and caudate putamen (0.70 from Bregma) were used for Scatchard analysis from untreated animals. Briefly, slides were allowed to reach room temperature and were incubated with 200 μl binding buffer (50 mM Tris-HCl, 5 % (w/v) BSA, pH 7.4 containing 50 μM PMSF) containing several concentrations of [$^3$H]CP55,940 (0.01, 0.1, 1, 10, 100 nM) in the absence (total binding) or 10 μM CP55,940 (non-specific binding) at room temperature for 120 min. After incubation, unbound ligand was removed by rinsing twice in wash buffer (50 mM Tris-HCl, 1 % (w/v) BSA, pH 7.4) at 4 °C for 10 min per wash, followed by a rapid dip in cold (4 °C) de-ionised water to remove salts. Sections were then either scraped from the slides with Whatman GF/C filters, placed in scintillation vials with 3 ml scintillation cocktail (Ultra Gold, PerkinElmer N.V./S.A. Belgium) and counted by liquid scintillation (Tri-Carb 1900CA liquid scintillation analyzer, Packard/ PerkinElmer N.V./S.A. Belgium). Alternatively sections were processed as for [$^3$H]CP55,940 autoradiography (see below). Transformation of data and calculation of the $K_d$ values were determined by a single-site binding equation (GraphPad Prism, San Diego, CA, U.S.A.).

Example C5: Cb-1 competition receptor binding in brain sections

For competition binding experiments, reaction buffers together with the incubation and wash times/temperatures were identical to the *in-situ* binding assay described previously. Coronal sections containing the nucleus accumbens and caudate putamen (0.70 from Bregma) were used from untreated animals. Seven concentrations of each compound ranging from 10 pM to 10 μM were assayed, with non-specific binding determined using 10 μM CP55,940 and total binding was determined using 10 nM [$^3$H]CP55,940. Each experiment was repeated three-times. $IC_{50}$ values were determined using GrapgPad Prism software (San Diego, CA, U.S.A.).

*Ex-vivo receptor binding in brain sections*

Three adjacent brain sections from the same treated animal (see *Feeding and receptor occupancy experiments*) were collected per slide through three different planes (0.70, -1.80, 3.30 mm rostral to Bregma). Reaction buffers and wash temperature/times were identical to the *in-situ* binding assay described previously. Incubation was restricted to 20 min at room temperature to minimize dissociation of drug from the receptor. Ex-vivo receptor labeling was expressed as the percentage of receptor labeling in corresponding brain regions of saline-treated animals. As only unoccupied

receptors remain available for the radioligand, ex-vivo receptor labeling is inversely proportional to the receptor occupancy by the in-vivo administered drug. Percentages of receptor occupancy by the drug administered to the animals correspond to 100 % minus the percentage of receptor labeling in the treated animal. The percentage of receptor occupancy was plotted against dosage, and the sigmoidal log dose-effect curve of best fit was calculated using nonlinear regression analysis using GraphPad Prism software (San Diego, CA, U.S.A.).

*[$^3$H]CP55,940 autoradiography*

After washing, the sections were then either rapidly dried under a stream of warm air before being placed in X-ray cassettes with slide mounted tritium micro-scale standards (RPA 501 and RPA 505; Amersham) and exposed to tritium-Hyperfilm (RPN 535B) for 10 weeks. The films were then developed in Kodak D 19 for 3 min at room temperature, washed and fixed. All autoradiograms were analyzed by computer densitometry (AIS system, Imaging Research Inc., Brock University, St. Catherines, Ontario, Canada). Optical densities in the anatomical regions of interest were transformed into levels of bound radioactivity (fmol/mg tissue equivalent) generated by the $^3$H-microscales.

pIC50 values for both the $CB_1$-competitive receptor binding experiments and the cAMP assay are provided in Table I *supra*. The results in this table are not given for the purpose of limiting the invention thereto but only to exemplify the useful pharmacological properties of all the compounds within the scope of formula (I).

Table I

| | Example C1 | Example C2 | Example C3 |
|---|---|---|---|
| Compound | Binding pIC50 | cAMP (antagonism) pIC50 | Food intake at 1 h ED50 (mg/kg) |
| 2 | 6.85 | | 2.69 |
| 3 | 6.8 | 6.75 | |
| 7 | 6.69 | 6.9 | |
| Intermediate 7 | 6.54 | 7.44 | 3.28 |
| 8 | 6.45 | 6.62 | |
| 10 | 6.28 | 6.38 | |
| 5 | 6.08 | 6.02 | |
| 9 | 6.05 | 5.79 | |
| 4 | 6.03 | 6.47 | |
| 11 | 5.67 | | |
| 13 | 5.61 | | |
| 12 | 5.53 | | |
| 6 | 5.27 | | |
| 1 | 5.05 | | >100 |

**Claims**

**1.** Use of a compound of formula (I) *infra* in the manufacture of a medicament for the treatment or prevention of obesity, psychiatric and neurological disorders, neuroinflammatory disorders, cognitive and memory disorders, psychosis, gastrointestinal disorders, addiction, asthma and liver cirrhosis

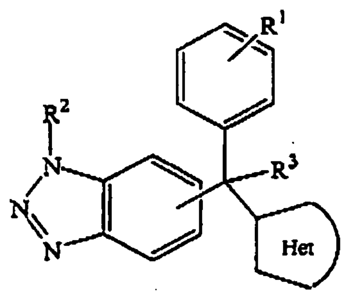

(I)

a pharmaceutically acceptable acid addition salts and stereochemically isomeric form thereof, wherein

$R^1$ is hydrogen, halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro;
$R^2$ is hydrogen; $C_{1-10}$alkyl optionally substituted with $Ar^1$, $C_{3-7}$cycloalkyl, hydroxyl or $C_{1-6}$alkyloxy; $Ar^1$; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenoxy optionally substituted with $Ar^2$; $C_{2-6}$alkynyloxy; pyrimidinyloxy; di($Ar^2$) methoxy; (1-$C_{1-4}$alkyl-4-piperidinyl)oxy; or $R^2$ is $C_{1-10}$alkyloxy optionally substituted with halo; hydroxyl; $C_{1-6}$alkyloxy; amino; mono- and di($C_{1-6}$alkyl)amino; trifluoromethyl; carboxyl; $C_{1-6}$alkyloxycarbonyl; $Ar^1$; $Ar^2$-O; $Ar^2$-S-; $C_{3-7}$cycloalkyl; 2,3-dhydro-1,4-benzodioxinyl; 1H-benzimidazolyl; $C_{1-4}$alkyl substituted 1H-benzimidazolyl; (1,1-biphenyl)-4-yl or with 2,3-dihydro-2-oxo-1H-benzimidazolyl;
$R^3$ is hydrogen, hydroxyl or $C_{1-6}$alkyl;
$Ar^1$ is phenyl, naphthalenyl, pyridinyl, aminopyridinyl, imidazolyl, triazolyl, thienyl, halothienyl, furanyl, $C_{1-6}$alkylfuranyl, halofuranyl, thiazolyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, nitro or mono- and di- ($C_{1-6}$alkyl)amino;
$Ar^2$ is phenyl, pyridinyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro; and
Het represents a monocyclic 5 or 6 membered partially saturated or aromatic heterocycle selected from furanyl, thienyl, pyrrolyl, oxazolyl, thiazolyl, imidazolyl, pyrazolyl, isoxazolyl, isothiazolyl, oxadiazolyl, triazolyl, thiadiazolyl, pyrimidinyl, pyridinyl, pyrazinyl, triazinyl, pyridazinyl, 2H-pyranyl or 4H-pyranyl wherein said heterocycle is optionally substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycubonyl-, carboxyl, formyl, (hydroxyimino)methyl, cyano, amino, mono- and di- ($C_{1-6}$alkyl)amino or nitro.

2. Use according to claim 1, wherein for the compound of formula (I)

$R^1$ is hydrogen, halo, trifluoromethyl, $C_{1-4}$alkyl, $C_{1-4}$alkyloxy- or $C_{1-4}$alkyloxycarbonyl-;
$R^2$ is phenyl, $C_{3-7}$cycloalkyl or $C_{1-6}$alkyl optionally substituted with $Ar^1$;
$Ar^1$ is phenyl or phenyl substituted with up to three halo substituents;
Het represents a monocyclic 5 or 6 membered partially saturated or aromatic heterocycle selected from thiazolyl, imidazolyl, triazolyl, pyrimidinyl or pyridinyl wherein said heterocycle is optionally substituted with $C_{1-4}$alkyl,

3. Use according to claims 1 or 2, wherein for the compound of formula (I)

Het is imidazolyl or 1,2,4-triazolyl;
$R^1$ is halo, $C_{1-4}$alkyl, $C_{1-4}$alkyloxy- or trifluoromethyl; and
$R^2$ is phenyl, $C_{3-7}$cycloalkyl or $C_{1-6}$alkyl optionally substituted with $Ar^1$.

4. Use according to claim 1 wherein the compound is selected from the group consisting of;

1H-Benzotriazole, (-)-6-[(4-chlorophenyl)-1H-1,2,4-triazol-1-ylmethyl]-1-cyclohexy-;
1H-Benzotriazole, 6-[(4-chlorophenyl)-1H-imidazol-1-ylmethyl]-1-cyclohexyl-;
1H-Benzotriazole, 6-[(4-chlorophenyl)-1H-1,2,4-triazol-1-ylmethyl]-1-(1-methylethyl)-;
1H-Benzotriazole, 6-[(4-chlorophenyl)-1H-1,2,4-triazol-1-ylmethyl]-1-cyclohexyl-;

1H-Benzotriazole, 1-butyl-6-[(4-chlorophenyl)-1H-1,2,4-triazol-1-ylmethyl]-;
1H-Benzotriazole, 6-[(4-chlorophenyl)-1H-imidazol-1-ylmethyl]-1-phenyl-;
1H-Benzotriaxole, 6-[(4-chlorophenyl)-1H-1,2,4-triazol-1-ylmethyl]-1-((4-chlorophenyl)methyl)-;
1H-Benzotriazole, 6-[phenyl-1H-imidazol-1-ylmethyl]-1-cyclohexyl-;
1H-Benzotriazole, 6-[(4-chlorophenyl)-1H-imidazol-1-ylmethyl]-1-(3-methylbutyl)-;
1H-Benzotriazole, 6-[(4-chlorophenyl)-1H-1,2,4-triazol-1-ylmethyl]-1-(phenylethyl)-;
1H-Benzotriazole, 6-[(4-chlorophenyl)-1H-1,2,4-triazol-1-ylmethyl]-1-(phenylmethyl)-;
1H-Benzotriazole, 6-[(4-chlorophenyl)-1H-imidazol-1-ylmethyl]-1-(1-methylethyl)-; and
1H-Benzotriazole, 6-[(4-chlorophenyl)-2-thiazolylmethyl]-1-methyl-; a stereoisomeric form thereof or a pharmaceutically acceptable acid or base addition salt thereof

**5.** A compound of formula (Ia)

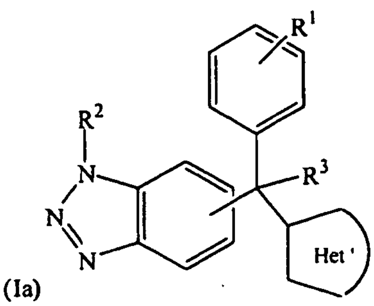

(Ia)

the pharmaceutically acceptable acid addition salts and stereoisomeric forms thereof, wherein

$R^1$ is hydrogen, halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro;
$R^2$ is hydrogen; $C_{1-10}$alkyl optionally substituted with $Ar^1$, $C_{3-7}$cycloalkyl, hydroxyl or $C_{1-6}$alkyloxy; $Ar^1$; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenoxy optionally substituted with $Ar^2$; $C_{2-6}$alkynyloxy; pyrimidinyloxy; di($Ar^2$)methoxy; (1-$C_{1-4}$alkyl-4-piperidinyl)oxy; or $R^2$ is $C_{1-10}$alkyloxy optionally substituted with halo; hydroxyl; $C_{1-6}$alkyloxy; amino; mono- and di($C_{1-6}$alkyl)amino; trifluoromethyl; carboxyl; $C_{1-6}$alkyloxycarbonyl; $Ar^1$; $Ar^2$-O-; $Ar^2$-S-; $C_{3-7}$cycloalkyl; 2,3-dihydro-1,4-benzodioxinyl; I H-benzimidazolyl; $C_{1-4}$alkyl substituted 1H-benzimidazolyl; (1,1-biphenyl)-4-yl or with 2,3-dihydro-2-oxo-1H-benzimidazolyl;
$R^3$ is hydrogen, hydroxyl or $C_{1-6}$alkyl;
$Ar^1$ is phenyl, naphthalenyl, pyridinyl, aminopyridinyl, imidazolyl, triazolyl, thienyl, halothienyl, furanyl, $C_{1-6}$alkylfuranyl, halofuranyl, thiazolyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, nitro or mono- and di- ($C_{1-6}$alkyl)amino;
$Ar^2$ is phenyl, pyridinyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, mono- and di- ($C_{1-6}$alkyl)amino or nitro; and
Het ' represents a monocyclic 5 or 6 membered partially saturated or aromatic heterocycle selected from thiazolyl, pyrimidinyl or pyridinyl wherein said heterocycle is optionally substituted with $C_{1-4}$alkyl;

provided that said compound of formula (Ia) does not represent
6-[(4-Chloro-phenyl)-pyridin-3-yl-methyl]-1-methyl-1H-benzotriazole or
6-[(4-Chloro-phenyl)-pyrimidin-5-yl-methyl]-1-methyl-1H-benzotriazole.

**6.** A compound according to claim 5 wherein;

$R^1$ is hydrogen, halo, trifluoromethyl, $C_{1-4}$alkyl, $C_{1-4}$alkyloxy- or $C_{1-4}$alkyloxycarbonyl;
$R^2$ is phenyl, $C_{3-7}$cycloalkyl or $C_{1-6}$alkyl optionally substituted with $Ar^1$;
$Ar^1$ is phenyl or phenyl substituted with up to 3 halo substituents;

**7.** A compound according to claims 5 or 6 wherein;

Het ' is thiazolyl;
$R^1$ is halo, $C_{1-4}$alkyl, $C_{1-4}$alkyloxy- or trifluoromethyl; and
$R^2$ is phenyl, $C_{3-7}$cycloalkyl or $C_{1-6}$alkyl optionally substituted with $Ar^1$.

**8.** A compound according to any one of claims 5 to 7 for use as a medicine.

**9.** Use of a compound according to any one of claims 5 to 7 in the manufacture of a medicament for the treatment of obesity, psychiatric and neurological disorders, neuroinflammatory disorders, cognitive and memory disorders, psychosis, gastrointestinal disorders, addiction, asthma and liver cirrhosis.

**10.** Use of a compound of formula (Ic) *infra* in the manufacture of a medicament for the treatment of obesity, psychiatric and neurological disorders, neuroinflammatory disorders, cognitive and memory disorders, psychosis, gastrointestinal disorders, addiction, asthma and liver cirrhosis

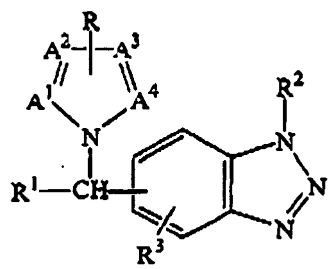

(Ic)

the pharmaceutically acceptable acid addition salts or a stereochemically isomeric form thereof, wherein $A^1=A^2-A^3=A^4$ is a bivalent radical having the formula

-CH=N-CH=CH- (a-1),

-CH=N-CH=N- (a2),

or

-CH=N-N=CH- (a3);

R is hydrogen or $C_{1-6}$alkyl;
$R^1$ is hydrogen, $C_{1-10}$alkyl, $C_{3-7}$cycloalkyl, $Ar^1$, $Ar^2$-$C_{1-6}$alkyl, $C_{2-6}$alkenyl or $C_{2-6}$alkynyl;
$R^2$ is hydrogen; $C_{1-10}$alkyl optionally substituted with $Ar^1$, $C_{3-7}$cycloalkyl, hydroxyl or $C_{1-6}$alkyloxy; $Ar^1$; $C_{2-6}$alkenyl; $C_{2-6}$alkynyl; $C_{3-7}$cycloalkyl; bicyclo[2.2.1]heptan-2-yl; 2,3-dihydro-1H-indenyl; 1,2,3,4-tetrahydronaphthalenyl; hydroxyl; $C_{2-6}$alkenoxy optionally substituted with $Ar^2$; $C_{2-6}$alkynyloxy; pyrimidinyloxy; di($Ar^2$)methoxy; (1-$C_{1-4}$alkyl-4-piperidinyl)oxy, or $R^2$ is $C_{1-10}$alkyloxy optionally substituted with halo; hydroxyl; $C_{1-6}$alkyloxy; amino; mono- and di($C_{1-6}$alkyl)amino; trifluoromethyl; carboxyl; $C_{1-6}$alkyloxycarbonyl; $Ar^1$; $Ar^2$-O-; $Ar^2$-S-; $C_{3-7}$cycloalkyl; 2,3-dihydro-1,4-benzodioxinyl; 1H-benzimidazolyl; $C_{1-4}$alkyl substituted 1H-benzimidazolyl; (1,1'-biphenyl)-4-yl or with 2,3-dihydro-2-oxo-1H-benzimidazolyl;
$R^3$ is hydrogen, nitro, amino, mono- and di($C_{1-6}$alkyl)amino, halo, $C_{1-6}$alkyl, hydroxyl or $C_{1-6}$alkyloxy;
$Ar^1$ is phenyl, substituted phenyl, naphthalenyl, pyridinyl, aminopyridinyl, imidazolyl, triazolyl, thienyl, halothienyl, furanyl, $C_{1-6}$alkylfuranyl, halofuranyl or thiazolyl; and
$Ar^2$ is phenyl, pyridinyl or phenyl substituted with up to 3 substituents each independently selected from halo, hydroxyl, hydroxymethyl, trifluoromethyl, $C_{1-6}$alkyl, $C_{1-6}$alkyloxy-, $C_{1-6}$alkyloxycarbonyl-, carboxyl, formyl, (hydroxyimino)methyl, cyano, amino, nitro or mono- and di- ($C_{1-6}$alkyl)amino;

**11.** Use of 1H-Benzotriazole, (-)-6-[(4-chlorophonyl)-1H-1,2,4-tiazol-1-ylmethyl]-1-cyclohexyl- in the manufacture of a medicament for treatment of obesity, psychiatric and neurological disorders, neuroinflammatory disorders, cognitive and memory disorders, psychosis, gastrointestinal disorders, addiction, asthma and liver cirrhosis.

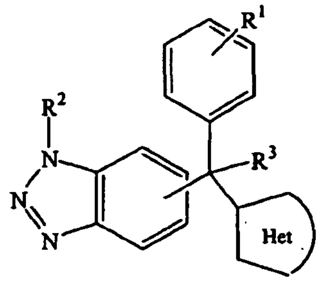

(I)

## Patentansprüche

1. Verwendung einer Verbindung der nachstehenden Formel (I) bei der Herstellung eines Arzneimittels zur Behandlung oder Prävention von Obesitas, psychiatrischen und neurologischen Störungen, neuroinflammatorischen Störungen, kognitiven Störungen, Gedächtnisstörungen, Psychose, gastrointestinalen Störungen, Sucht, Asthma und Leberzirrhose

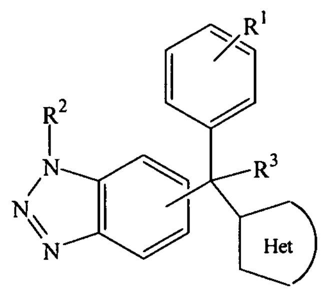

(I)

ein pharmazeutisch unbedenkliches Säureadditionssalz und eine stereochemisch isomere Form davon, worin
$R^1$ für Wasserstoff, Halogen, Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino)methyl, Cyano, Amino, Mono- und Di($C_{1-6}$-alkyl)amino oder Nitro steht;
$R^2$ für Wasserstoff; gegebenenfalls durch $Ar^1$, $C_{3-7}$-Cycloalkyl, Hydroxyl oder $C_{1-6}$-Alkyloxy substituiertes $C_{1-10}$-Alkyl; $Ar^1$; $C_{2-6}$-Alkenyl; $C_{2-6}$-Alkinyl; $C_{3-7}$-Cycloalkyl; Bicylo[2.2.1]heptan-2-yl; 2,3-Dihydro-1H-indenyl; 1,2,3,4-Tetrahydronaphthalinyl; Hydroxyl; gegebenenfalls durch $Ar^2$ substituiertes $C_{2-6}$-Alkenoxy; $C_{2-6}$-Alkinyloxy; Pyrimidinyloxy; Di($Ar^2$)methoxy; (1-$C_{1-4}$-Alkyl-4-piperidinyl)oxy steht oder $R^2$ für gegebenenfalls durch Halogen; Hydroxyl; $C_{1-6}$-Alkyloxy; Amino; Mono- und Di($C_{1-6}$-alkyl)amino; Trifluormethyl; Carboxyl; $C_{1-6}$-Alkyloxycarbonyl; $Ar^1$; $Ar^2$-O-; $Ar^2$-S-; $C_{3-7}$-Cycloalkyl; 2,3-Dihydro-1,4-benzodioxinyl; 1H-Benzimidazolyl; durch $C_{1-4}$-Alkyl substituiertes 1H-Benzimidazolyl; (1,1-Biphenyl)-4-yl oder durch 2,3-Dihydro-2-oxo-1H-benzimidazolyl substituiertes $C_{1-10}$-Alkyloxy steht;
$R^3$ für Wasserstoff, Hydroxyl oder $C_{1-6}$-Alkyl steht;
$Ar^1$ für Phenyl, Naphthalinyl, Pyridinyl, Aminopyridinyl, Imidazolyl, Triazolyl, Thienyl, Halogenthienyl, Furanyl, $C_{1-6}$-Alkylfuranyl, Halogenfuranyl, Thiazolyl oder Phenyl, das durch bis zu 3 jeweils unabhängig voneinander unter Halogen, Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino)methyl, Cyano, Amino, Nitro oder Mono- und Di($C_{1-6}$-alkyl)amino ausgewählte Substituenten substituiert ist, steht;
$Ar^2$ für Phenyl, Pyridinyl oder Phenyl, das durch bis zu 3 jeweils unabhängig voneinander unter Halogen, Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino) methyl, Cyano, Amino, Mono- und Di($C_{1-6}$-alkyl)amino oder Nitro ausgewählte Substituenten substituiert ist, steht und
Het für einen monocyclischen 5- oder 6-gliedrigen teilweise gesättigten oder aromatischen Heterocyclus steht, der unter Furanyl, Thienyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyrimidinyl, Pyridinyl, Pyrazinyl, Triazinyl, Pyridazinyl, 2H-Pyranyl oder 4H-Pyranyl ausgewählt ist und gegebenenfalls durch bis zu 3 jeweils unabhängig voneinander unter Halogen, Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino)methyl, Cyano, Amino, Mono- und Di($C_{1-6}$-alkyl)amino oder Nitro ausgewählte Substituenten substituiert ist.

**2.** Verwendung nach Anspruch 1, bei der für die Verbindung der Formel (I) $R^1$ für Wasserstoff, Halogen, Trifluormethyl, $C_{1-4}$-Alkyl, $C_{1-4}$-Alkyloxy- oder $C_{1-4}$-Alkyloxycarbonylsteht; $R^2$ für Phenyl, $C_{3-7}$-Cycloalkyl oder gegebenenfalls durch $Ar^1$ substituiertes $C_{1-6}$-Alkyl steht; $Ar^1$ für Phenyl oder durch bis zu drei Halogensubstituenten subtituiertes Phenyl steht; Het für einen monocyclischen 5- oder 6-gliedrigen teilweise gesättigten oder aromatischen Heterocyclus steht, der unter Thiazolyl, Imidazolyl, Triazolyl, Pyrimidinyl oder Pyridinyl ausgewählt ist und gegebenenfalls durch $C_{1-4}$-Alkyl substituiert ist.

**3.** Verwendung nach Anspruch 1 oder 2, bei der für die Verbindung der Formel (I) Het für Imidazolyl oder 1,2,4-Triazolyl steht; $R^1$ für Halogen, $C_{1-4}$-Alkyl, $C_{1-4}$-Alkyloxy- oder Trifluormethyl steht und $R^2$ für Phenyl, $C_{3-7}$-Cycloalkyl oder gegebenenfalls durch $Ar^1$ substituiertes $C_{1-6}$-Alkyl steht.

**4.** Verwendung nach Anspruch 1, bei der die Verbindung aus der Gruppe bestehend aus

(-)-6-[(4-Chlorphenyl)-1H-1,2,4-triazol-1-ylmethyl]-1-cyclohexyl-1H-benzotriazol;
6-[(4-Chlorphenyl)-1H-imidazol-1-ylmethyl]-1-cyclohexyl-1H-benzotriazol;
6-[(4-Chlorphenyl)-1H-1,2,4-triazol-1-ylmethyl]-1-(1-methylethyl)-1H-benzotriazol;
6-[(4-Chlorphenyl)-1H-1,2,4-triazol-1-ylmethyl]-1-cyclohexyl-1H-benzotriazol;
1-Butyl-6-[(4-chlorphenyl)-1H-1,2,4-triazol-1-ylmethyl]-1H-benzotriazol;
6-[(4-Chlorphenyl)-1H-imidazol-1-ylmethyl]-1-phenyl-1H-benzotriazol;
6-[(4-Chlorphenyl)-1H-1,2,4-triazol-1-ylmethyl]-1-((4-chlorphenyl)methyl)-1H-benzotriazol;
6-[Phenyl-1H-imidazol-1-ylmethyl]-1-cyclohexyl-1H-benzotriazol;
6-[(4-Chlorphenyl)-1H-imidazol-1-ylmethyl]-1-(3-methylbutyl)-1H-benzotriazol;
6-[(4-Chlorphenyl)-1H-1,2,4-triazol-1-ylmethyl]-1-(phenylethyl)-1H-benzotriazol;
6-[(4-Chlorphenyl)-1H-1,2,4-triazol-1-ylmethyl]-1-(phenylmethyl)-1H-benzotriazol;
6-[(4-Chlorphenyl)-1H-imidazol-1-ylmethyl]-1-(1-methylethyl)-1H-benzotriazol und
6-[(4-Chlorphenyl)-2-thiazolylmethyl]-1-methyl-1H-benzotriazol ausgewählt ist; eine stereoisomere Form davon oder ein pharmazeutisch unbedenkliches Säure- oder Basenadditionssalz davon.

**5.** Verbindung der Formel (Ia)

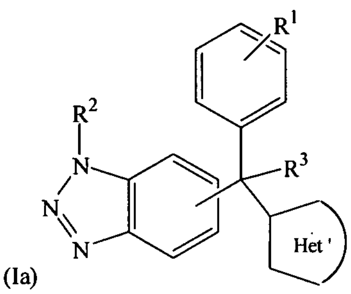

(Ia)

pharmazeutisch unbedenkliche Säureadditionssalze und stereoisomere Formen davon, worin

$R^1$ für Wasserstoff, Halogen, Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino) methyl, Cyano, Amino, Mono- und Di($C_{1-6}$-alkyl)amino oder Nitro steht;

$R^2$ für Wasserstoff; gegebenenfalls durch $Ar^1$, $C_{3-7}$-Cycloalkyl, Hydroxyl oder $C_{1-6}$-Alkyloxy substituiertes $C_{1-10}$-Alkyl; $Ar^1$; $C_{2-6}$-Alkenyl; $C_{2-6}$-Alkinyl; $C_{3-7}$-Cycloalkyl; Bicylo[2.2.1]heptan-2-yl; 2,3-Dihydro-1H-indenyl; 1,2,3,4-Tetrahydronaphthalinyl; Hydroxyl; gegebenenfalls durch $Ar^2$ substituiertes $C_{2-6}$-Alkenoxy; $C_{2-6}$-Alkinyloxy; Pyrimidinyloxy; Di($Ar^2$)methoxy; (1-$C_{1-4}$-Alkyl-4-piperidinyl)oxy steht oder $R^2$ für gegebenenfalls durch Halogen; Hydroxyl; $C_{1-6}$-Alkyloxy; Amino; Mono- und Di($C_{1-6}$-alkyl)amino; Trifluormethyl; Carboxyl; $C_{1-6}$-Alkyloxycarbonyl; $Ar^1$; $Ar^2$-O-; $Ar^2$-S-; $C_{3-7}$-Cycloalkyl; 2,3-Dihydro-1,4-benzodioxinyl; 1H-Benzimidazolyl; durch $C_{1-4}$-Alkyl substituiertes 1H-Benzimidazolyl; (1,1-Biphenyl)-4-yl oder durch 2,3-Dihydro-2-oxo-1H-benzimidazolyl substituiertes $C_{1-10}$-Alkyloxy steht;

$R^3$ für Wasserstoff, Hydroxyl oder $C_{1-6}$-Alkyl steht;

$Ar^1$ für Phenyl, Naphthalinyl, Pyridinyl, Aminopyridinyl, Imidazolyl, Triazolyl, Thienyl, Halogenthienyl, Furanyl, $C_{1-6}$-Alkylfuranyl, Halogenfuranyl, Thiazolyl oder Phenyl, das durch bis zu 3 jeweils unabhängig voneinander unter Halogen,

Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino)methyl, Cyano, Amino, Nitro oder Mono- und Di($C_{1-6}$-alkyl)amino ausgewählte Substituenten substituiert ist, steht;
$Ar^2$ für Phenyl, Pyridinyl oder Phenyl, das durch bis zu 3 jeweils unabhängig voneinander unter Halogen, Hydroxyl, Hydroxymethyl, Trifluormethyl, $C_{1-6}$-Alkyl, $C_{1-6}$-Alkyloxy-, $C_{1-6}$-Alkyloxycarbonyl-, Carboxyl, Formyl, (Hydroxyimino) methyl, Cyano, Amino, Mono- und Di($C_{1-6}$-alkyl)amino oder Nitro ausgewählte Substituenten substituiert ist, steht und
Het' für einen monocyclischen 5- oder 6-gliedrigen teilweise gesättigten oder aromatischen Heterocyclus steht, der unter Thiazolyl, Pyrimidinyl oder Pyridinyl ausgewählt ist und gegebenenfalls durch $C_{1-4}$-Alkyl substituiert ist;
mit der Maßgabe, daß die Verbindung der Formel (Ia) nicht
6-[(4-Chlorphenyl)pyridin-3-ylmethyl]-1-methyl-1H-benzotriazol oder
6-[(4-Chlorphenyl)pyrimidin-5-ylmethyl]-1-methyl-1H-benzotriazol
darstellt.

**6.** Verbindung nach Anpruch 5, worin
$R^1$ für Wasserstoff, Halogen, Trifluormethyl, $C_{1-4}$-Alkyl, $C_{1-4}$-Alkyloxy- oder $C_{1-4}$-Alkyloxycarbonylsteht;
$R^2$ für Phenyl, $C_{3-7}$-Cycloalkyl oder gegebenenfalls durch $Ar^1$ substituiertes $C_{1-6}$-Alkyl steht;
$Ar^1$ für Phenyl oder durch bis zu drei Halogensubstituenten subtituiertes Phenyl steht.

**7.** Verbindung nach Anpruch 5 oder 6, worin
Het' für Thiazolyl steht;
$R^1$ für Halogen, $C_{1-4}$-Alkyl, $C_{1-4}$-Alkyloxy- oder Trifluormethyl steht;
$R^2$ für Phenyl, $C_{3-7}$-Cycloalkyl oder gegebenenfalls durch $Ar^1$ substituiertes $C_{1-6}$-Alkyl steht.

**8.** Verbindung nach einem der Ansprüche 5 bis 7 zur Verwendung als Medizin.

**9.** Verwendung einer Verbindung nach einem der Ansprüche 5 bis 7 bei der Herstellung eines Arzneimittels zur Behandlung von Obesitas, psychiatrischen und neurologischen Störungen, neuroinflammatorischen Störungen, kognitiven Störungen, Gedächtnisstörungen, Psychose, gastrointestinalen Störungen, Sucht, Asthma und Leberzirrhose.

**10.** Verwendung einer Verbindung der nachstehenden Formel (Ic) bei der Herstellung eines Arzneimittels zur Behandlung von Obesitas, psychiatrischen und neurologischen Störungen, neuroinflammatorischen Störungen, kognitiven Störungen, Gedächtnisstörungen, Psychose, gastrointestinalen Störungen, Sucht, Asthma und Leberzirrhose

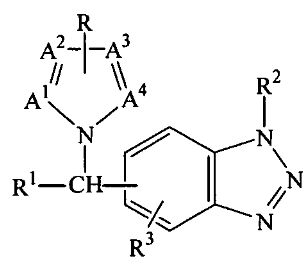

(Ic)

die pharmazeutisch unbedenklichen Säureadditionssalze oder eine stereochemisch isomere Form davon, worin $A^1$=$A^2$-$A^3$=$A^4$ für einen zweiwertigen Rest der Formel

-CH=N-CH=CH- (a-1),

-CH=N-CH=N- (a-2)

oder

-CH=N-N=CH- (a3)

steht;

R für Wasserstoff oder $C_{1-6}$-Alkyl steht;
$R^1$ für Wasserstoff, $C_{1-10}$-Alkyl, $C_{3-7}$-Cycloalkyl, $Ar^1$, $Ar^2$-$C_{1-6}$-Alkyl, $C_{2-6}$-Alkenyl oder $C_{2-6}$-Alkinyl steht;
$R^2$ für Wasserstoff; gegebenenfalls durch $Ar^1$, $C_{3-7}$-Cycloalkyl, Hydroxyl oder $C_{1-6}$-Alkyloxy substituiertes $C_{1-10}$-Alkyl; $Ar^1$; $C_{2-6}$-Alkenyl; $C_{2-6}$-Alkinyl; $C_{3-7}$-Cycloalkyl; Bicylo[2.2.1]heptan-2-yl; 2,3-Dihydro-1H-indenyl; 1,2,3,4-Tetrahydronaphthalinyl; Hydroxyl; gegebenenfalls durch $Ar^2$ substituiertes $C_{2-6}$-Alkenoxy; $C_{2-6}$-Alkinyloxy; Pyrimidinyloxy; Di($Ar^2$)methoxy; (1-$C_{1-4}$-Alkyl-4-piperidinyl)oxy steht oder $R^2$ für gegebenenfalls durch Halogen; Hydroxyl; $C_{1-6}$-Alkyloxy; Amino; Mono- und Di($C_{1-6}$-alkyl)amino; Trifluormethyl; Carboxyl; $C_{1-6}$-Alkyloxycarbonyl; $Ar^1$; $Ar^2$-O-; $Ar^2$-S-; $C_{3-7}$-Cycloalkyl; 2,3-Dihydro-1,4-benzodioxinyl; 1H-Benzimidazolyl; durch $C_{1-4}$-Alkyl substituiertes 1H-Benzimidazolyl; (1,1-Biphenyl)-4-yl oder durch 2,3-Dihydro-2-oxo-1H-benzimidazolyl substituiertes $C_{1-10}$-Alkyloxy steht;
$R^3$ für Wasserstoff, Nitro, Amino, Mono- und Di($C_{1-6}$-alkyl)amino, Halogen, $C_{1-6}$-Alkyl, Hydroxyl oder $C_{1-6}$-Alkyloxy steht;
$Ar^1$ für Phenyl, substituiertes Phenyl, Naphthalinyl, Pyridinyl, Aminopyridinyl, Imidazolyl, Triazolyl, Thienyl, Halogenthienyl, Furanyl, $C_{1-6}$-Alkylfuranyl, Halogenfuranyl oder Thiazolyl steht;
$Ar^2$ für Phenyl, Pyridinyl oder Phenyl, das durch bis zu 3 jeweils unabhängig voneinander unter Halogen, Hydroxyl, Hydroxymethyl, Triflucrmethyl, $C_{1-6}$-Alkyl, $C_{1-6}$-Alkyloxy-, $C_{1-6}$-Alkyloxycarbonyl-, Carboxyl, Formyl, (Hydroxyimino) methyl, Cyano, Amino, Nitro oder Mono- und Di($C_{1-6}$-alkyl)amino ausgewählte Substituenten substituiert ist, steht.

**11.** Verwendung von (-)-6-[(4-Chlorphenyl)-1H-1,2,4-triazol-1-ylmethyl]-1-cyclohexyl-1H-benzotriazol bei der Herstellung eines Arzneimittels zur Behandlung von Obesitas, psychiatrischen und neurologischen Störungen, neuroinflammatorischen Störungen, kognitiven Störungen, Gedächtnisstörungen, Psychose, gastrointestinalen Störungen, Sucht, Asthma und Leberzirrhose.

## Revendications

**1.** Utilisation d'un composé de formule (I) *infra* dans la fabrication d'un médicament destiné au traitement ou à la prévention de troubles d'obésité, psychiatriques et neurologiques, de troubles neuroinflammatoires, de troubles cognitifs et mnésiques, de la psychose, de troubles gastro-intestinaux, de la dépendance, de l'asthme et de la cirrhose du foie

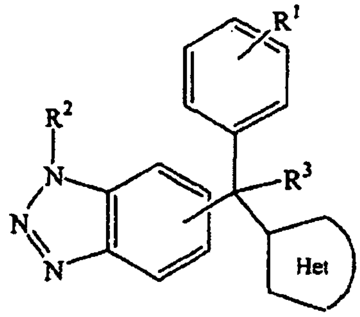

(I)

un sel d'addition d'acide pharmaceutiquement acceptable et une forme stéréochimiquement isomère de celui-ci, dans laquelle
$R^1$ est hydrogène, halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, mono- et di-($C_{1-6}$alkyl)amino ou nitro ;
$R^2$ est hydrogène ; $C_{1-10}$alkyle éventuellement substitué par $Ar^1$, $C_{3-7}$cycloalkyle, hydroxy ou $C_{1-6}$alkyloxy ; $Ar^1$ ; $C_{2-6}$alcényle ; $C_{2-6}$alcynyle ; $C_{3-7}$cycloalkyle ; bicyclo[2,2,1]heptan-2-yle ; 2,3-dihydro-1H-indényle ; 1,2,3,4-tétrahydronaphtalényle ; hydroxy ; $C_{2-6}$alcénoxy éventuellement substitué par $Ar^2$ ; $C_{2-6}$alcynyloxy ; pyrimidinyloxy ; di($Ar^2$)méthoxy ; (1-$C_{1-4}$alkyl-4-pipéridinyl)oxy ; ou $R^2$ est $C_{1-10}$alkyloxy éventuellement substitué par halogéno ; hydroxy ; $C_{1-6}$alkyloxy ; amino ; mono- et di($C_{1-6}$alkyl)amino ; trifluorométhyle ; carboxy ; $C_{1-6}$alkyloxycarbonyle ; $Ar^1$ ; $Ar^2$-O- ; $Ar^2$-S- ; $C_{3-7}$cycloalkyle ; 2,3-dihydro-1,4-benzodioxinyle ; 1H-benzimidazolyle ; 1H-benzimidazolyle substitué par $C_{1-4}$alkyle ; (1,1-biphényl)-4-yle ou par 2,3-dihydro-2-oxo-1H-benzimidazolyle ;
$R^3$ est hydrogène, hydroxy ou $C_{1-6}$alkyle ;
$Ar^1$ est phényle, naphtalényle, pyridinyle, aminopyridinyle, imidazolyle, triazolyle, thiényle, halogénothiényle, fura-

nyle, $C_{1-6}$alkylfuranyle, halogénofuranyle, thiazolyle ou phényle substitué par jusqu'à 3 substituants choisis chacun indépendamment parmi halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, nitro ou mono- et di-($C_{1-6}$alkyl) amino ;

$Ar^2$ est phényle, pyridinyle ou phényle substitué par jusqu'à 3 substituants choisis chacun indépendamment parmi halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, mono- et di-($C_{1-6}$alkyl)amino ou nitro ; et

Het représente un hétérocycle monocyclique partiellement saturé ou aromatique à 5 ou 6 chaînons choisi parmi furanyle, thiényle, pyrrolyle, oxazolyle, thiazolyle, imidazolyle, pyrazolyle, isoxazolyle, isothiazolyle, oxadiazolyle, triazolyle, thiadiazolyle, pyrimidinyle, pyridinyle, pyrazinyle, triazinyle, pyridazinyle, 2H-pyranyle ou 4H-pyranyle où ledit hétérocycle est éventuellement substitué par jusqu'à 3 substituants choisis chacun indépendamment parmi halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, mono- et di-($C_{1-6}$alkyl)amino ou nitro.

**2.** Utilisation selon la revendication 1, **caractérisée en ce que** pour le composé de formule (I)
$R^1$ est hydrogène, halogéno, trifluorométhyle, $C_{1-4}$alkyle, $C_{1-4}$alkyloxy- ou $C_{1-4}$alkyloxycarbonyl- ;
$R^2$ est phényle, $C_{3-7}$cycloalkyle ou $C_{1-6}$alkyle éventuellement substitué par $Ar^1$;
$Ar^1$ est phényle ou phényle substitué par jusqu'à trois substituants halogéno ;
Het représente un hétérocycle monocyclique partiellement saturé ou aromatique à 5 ou 6 chaînons choisi parmi thiazolyle, imidazolyle, triazolyle, pyrimidinyle ou pyridinyle où ledit hétérocycle est éventuellement substitué par $C_{1-4}$alkyle.

**3.** Utilisation selon la revendication 1 ou 2, **caractérisée en ce que** pour le composé de formule (I)
Het est imidazolyle ou 1,2,4-triazolyle ;
$R^1$ est halogéno, $C_{1-4}$alkyle, $C_{1-4}$alkyloxy- ou trifluorométhyle ; et
$R^2$ est phényle, $C_{3-7}$cycloalkyle ou $C_{1-6}$alkyle éventuellement substitué par $Ar^1$.

**4.** Utilisation selon la revendication 1, **caractérisée en ce que** le composé est choisi dans le groupe constitué de ;

1H-benzotriazole, (-)-6-[(4-chlorophényl)-1H-1,2,4-triazol-1-ylméthyl]-1-cyclohexyl- ;
1H-benzotriazole, 6-[(4-chlorophényl)-1H-imidazol-1-ylméthyl]-1-cyclohexyl- ;
1H-benzotriazole, 6-[(4-chlorophényl)-1H-1,2,4-triazol-1-ylméthyl]-1-(1-méthyléthyl)- ;
1H-benzotriazole, 6-[(4-chlorophényl)-1H-1,2,4-triazol-1-ylméthyl]-1-cyclohexyl- ;
1H-benzotriazole, 1-butyl-6-[(4-chlorophényl)-1H-1,2,4-triazol-1-ylméthyl]- ;
1H-benzotriazole, 6-[(4-chlorophényl)-1H-imidazol-1-ylméthyl]-1-phényle ;
1H-benzotriazole, 6-[(4-chlorophényl)-1H-1,2,4-triazol-1-ylméthyl]-1-((4-chlorophényl)méthyl)- ;
1H-benzotriazole, 6-[phényl-1H-imidazol-1-ylméthyl]-1-cyclohexyl- ;
1H-benzotriazole, 6-[(4-chlorophényl)-1H-imidazol-1-ylméthyl]-1-(3-méthylbutyl)- ;
1H-benzotriazole, 6-[(4-chlorophényl)-1H-1,2,4-triazol-1-ylméthyl]-1-(phényléthyl)- ;
1H-benzotriazole, 6-[(4-chlorophényl)-1H-1,2,4-triazol-1-ylméthyl]-1-(phénylméthyl)- ;
1H-benzotriazole, 6-[(4-chlorophényl)-1H-imidazol-1-ylméthyl]-1-(1-méthyléthyl)- ; et
1H-benzotriazole, 6-[(4-chlorophényl)-2-thiazolylméthyl]-1-méthyl- ; une forme stéréoisomère de ceux-ci ou un sel d'addition d'acide ou de base pharmaceutiquement acceptable de ceux-ci.

**5.** Composé de formule (Ia)

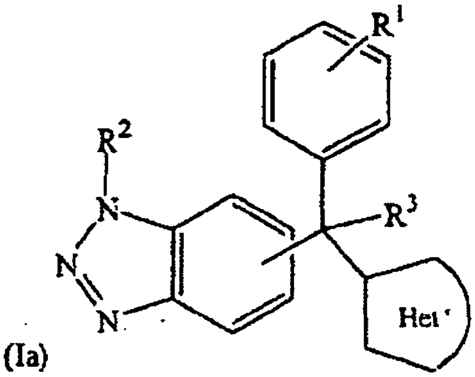

(Ia)

les sels d'addition d'acides pharmaceutiquement acceptables et les formes stéréoisomères de celui-ci, dans laquelle $R^1$ est hydrogène, halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, mono- et di-($C_{1-6}$alkyl)amino ou nitro ;
$R^2$ est hydrogène ; $C_{1-10}$alkyle éventuellement substitué par $Ar^1$, $C_{3-7}$cycloalkyle, hydroxy ou $C_{1-6}$alkyloxy ; $Ar^1$ ; $C_{2-6}$alcényle ; $C_{2-6}$alcynyle ; $C_{3-7}$ cycloalkyle ; bicyclo[2,2,1]heptan-2-yle ; 2,3-dihydro-1H-indényle ; 1,2,3,4-tétrahydronaphtalényle ; hydroxy ; $C_{2-6}$alcénoxy éventuellement substitué par $Ar^2$ ; $C_{2-6}$alcynyloxy ; pyrimidinyloxy ; di($Ar^2$)méthoxy ; (1-$C_{1-4}$alkyl-4-pipéridinyl)oxy ; ou $R^2$ est $C_{1-10}$alkyloxy éventuellement substitué par halogéno ; hydroxy ; $C_{1-6}$alkyloxy ; amino ; mono- et di($C_{1-6}$alkyl)amino ; trifluorométhyle ; carboxy ; $C_{1-6}$alkyloxycarbonyle ; $Ar^1$ ; $Ar^2$-O- ; $Ar^2$-S- ; $C_{3-7}$cycloalkyle ; 2,3-dihydro-1,4-benzodioxinyle ; 1H-benzimidazolyle ; 1H-benzimidazolyle substitué par $C_{1-4}$alkyle ; (1,1-biphényl)-4-yle ou par 2,3-dihydro-2-oxo-1H-benzimidazolyle ;
$R^3$ est hydrogène, hydroxy ou $C_{1-6}$alkyle ;
$Ar^1$ est phényle, naphtalényle, pyridinyle, aminopyridinyle, imidazolyle, triazolyle, thiényle, halogénothiényle, furanyle, $C_{1-6}$alkylfuranyle, halogénofuranyle, thiazolyle ou phényle substitué par jusqu'à 3 substituants choisis chacun indépendamment parmi halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, nitro ou mono- et di-($C_{1-6}$alkyl) amino ;
$Ar^2$ est phényle, pyridinyle ou phényle substitué par jusqu'à 3 substituants choisis chacun indépendamment parmi halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, mono- et di-($C_{1-6}$alkyl)amino ou nitro ; et
Het' représente un hétérocycle monocyclique partiellement saturé ou aromatique à 5 ou 6 chaînons choisi parmi thiazolyle, pyrimidinyle ou pyridinyle où ledit hétérocycle est éventuellement substitué par $C_{1-4}$alkyle ;
à condition que ledit composé de formule (Ia) ne représente pas
le 6-[(4-chlorophényl)-pyridin-3-yl-méthyl]-1-méthyl-1H-benzotriazole ou
le 6-[(4-chlorophényl)-pyrimidin-5-yl-méthyl]-1-méthyl-1H-benzotriazole.

**6.** Composé selon la revendication 5, **caractérisé en ce que ;**
$R^1$ est hydrogène, halogéno, trifluorométhyle, $C_{1-4}$alkyle, $C_{1-4}$alkyloxy- ou $C_{1-4}$alkyloxycarbonyle ;
$R^2$ est phényle, $C_{3-7}$cycloalkyle ou $C_{1-6}$alkyle éventuellement substitué par $Ar^1$ ;
$Ar^1$ est phényle ou phényle substitué par jusqu'à 3 substituants halogéno.

**7.** Composé selon la revendication 5 ou 6, **caractérisé en ce que ;**
Het' est thiazolyle ;
$R^1$ est halogéno, $C_{1-4}$alkyle, $C_{1-4}$alkyloxy- ou trifluorométhyle ; et
$R^2$ est phényle, $C_{3-7}$cycloalkyle ou $C_{1-6}$alkyle éventuellement substitué par $Ar^1$.

**8.** Composé selon l'une quelconque des revendications 5 à 7, destiné à être utilisé comme médicament.

**9.** Utilisation d'un composé selon l'une quelconque des revendications 5 à 7, dans la fabrication d'un médicament destiné au traitement de troubles d'obésité, psychiatriques, et neurologiques, de troubles neuroinflammatoires, de troubles cognitifs et mnésiques, de la psychose, de troubles gastro-intestinaux, de la dépendance, de l'asthme et de la cirrhose du foie.

**10.** Utilisation d'un composé de formule (Ic) *infra* dans la fabrication d'un médicament destiné au traitement de troubles d'obésité, psychiatriques et neurologiques, de troubles neuroinflammatoires, de troubles cognitifs et mnésiques, de la psychose, de troubles gastro-intestinaux, de la dépendance, de l'asthme et de la cirrhose du foie.

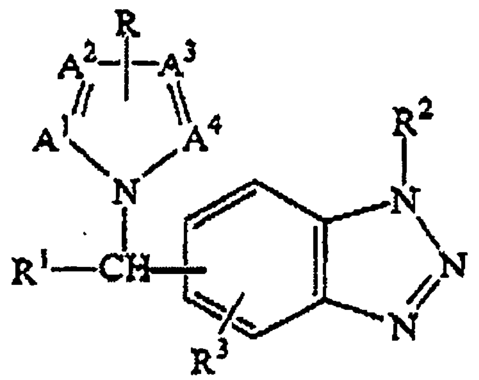

(Ic)

les sels d'addition d'acides pharmaceutiquement acceptables ou une forme stéréochimiquement isomère de celui-ci, dans laquelle $A^1=A^2-A^3=A^4$ est un radical bivalent ayant la formule

-CH=N-CH=CH- (a-1),

-CH=N-CH=N- (a2),

ou

-CH=N-N=CH- (a3) ;

R est hydrogène ou $C_{1-6}$alkyle ;
$R^1$ est hydrogène, $C_{1-10}$alkyle, $C_{3-7}$cycloalkyle, $Ar^1$, $Ar^2$-$C_{1-6}$alkyle, $C_{2-6}$alcényle ou $C_{2-6}$alcynyle ;
$R^2$ est hydrogène ; $C_{1-10}$alkyle éventuellement substitué par $Ar^1$, $C_{3-7}$cycloalkyle, hydroxy ou $C_{1-6}$alkyloxy ; $Ar^1$ ; $C_{2-6}$alcényle ; $C_{2-6}$alcynyle ; $C_{3-7}$cycloalkyle ; bicyclo[2,2,1]heptan-2-yle ; 2,3-dihydro-1H-indényle ; 1,2,3,4-tétrahydronaphtalényle ; hydroxy ; $C_{2-6}$alcénoxy éventuellement substitué par $Ar^2$ ; $C_{2-6}$alcynyloxy ; pyrimidinyloxy ; di($Ar^2$)méthoxy ; (1-$C_{1-4}$alkyl-4-pipéridinyl)oxy ; ou $R^2$ est $C_{1-10}$alkyloxy éventuellement substitué par halogéno ; hydroxy ; $C_{1-6}$alkyloxy ; amino ; mono- et di($C_{1-6}$alkyl)amino ; trifluorométhyle ; carboxy ; $C_{1-6}$alkyloxycarbonyle ; $Ar^1$ ; $Ar^2$-O- ; $Ar^2$-S- ; $C_{3-7}$cycloalkyle ; 2,3-dihydro-1,4-benzodioxinyle ; 1H-benzimidazolyle ; 1H-benzimidazolyle substitué par $C_{1-4}$alkyle .; (1,1'-biphényl)-4-yle ou par 2,3-dihydro-2-oxo-1H-benzimidazolyle ;
$R^3$ est hydrogène, nitro, amino, mono- et di($C_{1-6}$alkyl)amino, halogéno, $C_{1-6}$alkyle, hydroxy ou $C_{1-6}$alkyloxy ;
$Ar^1$ est phényle, phényle substitué, naphtalényle, pyridinyle, aminopyridinyle, imidazolyle, triazolyle, thiényle, halogénothiényle, furanyle, $C_{1-6}$alkylfuranyle, halogénofuranyle ou thiazolyle ; et
$Ar^2$ est phényle, pyridinyle ou phényle substitué par jusqu'à 3 substituants choisis chacun indépendamment parmi halogéno, hydroxy, hydroxyméthyle, trifluorométhyle, $C_{1-6}$alkyle, $C_{1-6}$alkyloxy-, $C_{1-6}$alkyloxycarbonyl-, carboxy, formyle, (hydroxyimino)méthyle, cyano, amino, nitro ou mono- et di-($C_{1-6}$alkyl)amino.

**11.** Utilisation de 1H-benzotriazole, (-)-6-[(4-chlorophényl)-1H-1,2,4-triazol-1-ylméthyl]-1-cyclohexyl- dans la fabrication d'un médicament destiné au traitement de troubles d'obésité, psychiatriques et neurologiques, de troubles neuroinflammatoires, de troubles cognitifs et mnésiques, de la psychose, de troubles gastro-intestinaux, de la dépendance, de l'asthme et de la cirrhose du foie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 293978 A


### Non-patent literature cited in the description


- **Venet M. et al.** *Actualités de chimie thérapeutique,* 1997, vol. 23, 239-246
- **Lidström P. et al.** *Nuclear Medicine & Biology,* 1998, vol. 25, 497-501
- *Current Opinion in Drug Discovery & Development,* 2004, vol. 7 (4), 498-506
- **Jerry March.** Advanced Organic Chemistry. 9-48